# EUROPEAN PATENT APPLICATION

(11) **EP 2 977 469 A1**
(43) Date of publication of application: **27.01.2016**
(21) Application number: 15182776.3
(22) Date of filing: 28.08.2009
(51) Int. Cl.: C12Q 1/68, G01N 33/68, A61Q 19/00

(54) **DETERMINING AGE RANGES OF SKIN SAMPLES**

(30) Priority: 28.08.2008 US 92687 P
(62) Divisional of application: 09810644.6
(71) Applicant: Dermtech International, La Jolla, CA 92037 (US)
(72) Inventor: CHANG, Sherman, J, San Diego, CA 92131 (US); NAUGHTON, Gail, K, San Diego, CA92101 (US)
(74) Representative: Ali, Suleman

(57) **Abstract**

A method for determining the age range of a skin sample comprising:
a) providing a skin sample comprising nucleic acids expressed by genes in a classifier comprising one or more young genes and one or more old genes;
b) determining expression levels of the nucleic acids expressed from at least one young gene and at least one old gene in the classifier by assaying the nucleic acids in the skin sample; and
c) characterizing the age range of the skin sample based on the expression levels.

## Description

### FIELD OF THE INVENTION

The present invention relates generally to skin sampling and more specifically to methods of characterizing skin based on an age index.

### BACKGROUND INFORMATION

Aging is the accumulation of changes in an organism or object over time. Aging in humans refers to a multidimensional process of physical, psychological, and social change. Some dimensions of aging grow and expand over time, while others decline. Reaction time, for example, may slow with age, while knowledge of world events and wisdom may expand. Research shows that even late in life potential exists for physical, mental, and social growth and development. Aging is an important part of all human societies reflecting the biological changes that occur, but also reflecting cultural and societal conventions.

As far as mammals go, humans are essentially hairless; that is, most of the skin of the human body can be seen without interference from hair. The skin is thus exposed to whatever insults (natural and man-made) the environment harbors. Since it was first understood that the sun caused erythema, people have taken measures to avoid its "harmful rays." A century ago, in Elizabethan England, it was the fashion to avoid the sun at all costs. Yet the skin of those Elizabethans still wrinkled and displayed other signs of chronological aging.

Human skin is a complex organ which extends over the entire body. There are different types of skin at different portions of the body; for example, facial skin is different from that of the scalp, and even the skin on the front (palm) of the hand is different than that on the back of the hand. Although the type of skin can vary over a person's body, skin is generally composed of two main layers of tissue. The epidermis or cuticle, the outermost layer, is composed of superficial layers (from the outside in: stratum corneum, stratum lucidem, and stratum granulosum) and deep layers (stratum spinosum and stratum basale). The dermis, cutis vera, or the true skin, is composed of a papillary layer above and a reticular layer below.

Since ancient times, a variety of substances have been applied to the skin to improve its appearance, generally by affecting the outermost layer of the skin, or to treat a skin ailment, generally by affecting the true skin. More recently, efforts have been made to rejuvenate the skin and reclaim the elasticity and suppleness lost from exposure to sunlight (UV radiation) and weather.

There is a difference between the physiology of chronologically-aged or intrinsically-aged skin (old skin) in comparison with that of photoaged skin (i.e., skin that appears old due to damage from solar UV irradiation). Old skin typically maintains a smooth and unblemished appearance, in comparison with the leathery, blotchy, and often deep wrinkling of photoaged skin. The epidermis of old skin is typically thinner than normal, whereas that of photoaged aged skin is typically thicker than normal (acanthotic) and atrophies over time. Photoaged skin typically has a large Grenz zone (a wide band of eosinophilic material just beneath the epidermis, and collagen formation and structures indicative of wound healing) which is absent from chronologically-aged skin. See also N. A. Fenske and C. W. Lober, "Structural and functional changes of normal aging skin," J. Am. Acad. Dermatol., 15:571-585 (1986).

In biology, "senescence" is the state or process of aging. "Cellular senescence" is a phenomenon where isolated cells demonstrate a limited ability to divide in culture (*i.e.,* the Hayflick Limit, discovered by Leonard Hayflick in 1965), while "Organismal senescence" is the aging of organisms. After a period of near perfect renewal (in humans, between about 20 and 35 years of age), organismal senescence is characterized by the declining ability to respond to stress, increasing homeostatic imbalance and increased risk of disease. This irreversible series of changes inevitably ends in death. Some researchers (specifically biogerontologists) are treating aging as a disease. As genes that have an effect on aging are discovered, aging is increasingly being regarded in a similar fashion to other genetic conditions, *i.e.,* potentially "treatable."

Clearly there is a need for further development of technology that will enable physicians to develop a skin age index to predict aging of individuals' skin.

### SUMMARY OF THE INVENTION

The present invention is based, in part, on the discovery that analysis of nucleic acids or protein products from specific genes can be used to distinguish or categorize skin samples of individuals. The methods provide valuable genetic information based on DNA, RNA, or protein products obtained therefrom, for example.

In one embodiment, the method involves use of a non-invasive approach for recovering nucleic acids such as DNA or RNA or proteins from the surface of skin via a tape stripping procedure that permits a direct quantitative and qualitative assessment of biomarkers. Although tape-harvested nucleic acid and protein products are shown to be comparable in quality and utility to recovering such molecules by biopsy, the non-invasive method provides information regarding cells of the outermost layers of the skin that may not be obtained using biopsy samples. Finally, the non-invasive method is far less traumatic than a biopsy, although the invention does not exclude the use of such invasive methods for skin sampling.

Thus, the method is used to capture cells on the skin of individuals for determination of the appropriate age range. Nucleic acid molecules obtained from skin are analyzed in order to characterize the skin sample as being young or old. In one embodiment, a nucleic acid molecule is amplified prior to analysis. Secondary outcomes could include tests for diagnosis and prognosis of a variety of symptoms of photoaging and/or chronoaging of skin, and to predict a therapeutic or cosmetic regimen for treating the skin. In another embodiment, the skin cells are lysed to extract one or more proteins, which are then quantitated to compare with gene products of the genes listed in Table 1, Table 2, Table 3, or any combination thereof, for example, however, the combination must include at least one "young" gene and at least one "old" gene. It should be understood that the methods of the invention are not limited to non-invasive techniques as described herein for obtaining skin samples. For example, but not by limitation, one of skill in the art would know other techniques for obtaining a skin sample such as scraping of the skin, biopsy, suction, blowing and other techniques. As described herein, non-invasive tape stripping, as described in U.S. Pat. No. 6,949,338, incorporated herein by reference, is an illustrative example for obtaining a skin sample.

In another embodiment, the methods involve detection of one or more mutations in the nucleic acid sequence of the nucleic acid molecule obtained from the skin. Such mutations may be a substitution, a deletion, and/or an insertion of the nucleic acid sequence that results in symptoms associated with chronoaging and/or photoaging of skin from the subject from which the skin sample is obtained.

In one embodiment, the nucleic acid analyzed is any one or more of those listed in Table 1, Table 2, Table 3, or any combination thereof. Accordingly, provided herein is a method for determining the age range of a skin sample of a subject, including obtaining a nucleic acid or protein by tape stripping or biopsy of the skin or a skin lesion, for example, from the subject, and analyzing the nucleic acid as compared to the nucleic acids or protein products thereof listed in Table 1, Table 2, Table 3, or any combination thereof. In this method, at least one nucleic acid molecule whose expression is informative of an appropriate age range of the skin is detected in the sample.

The non-invasive methods of the invention involve applying an adhesive tape to a target area of skin in a manner sufficient to isolate a sample adhering to the adhesive tape, wherein the sample includes nucleic acid molecules and/or proteins. Typically, at least one nucleic acid molecule or protein whose expression is informative of the age or age range of the skin in the sample. The method of characterizing skin using tape stripping has a number of applications, such as the following: (i) age classification of the skin; (ii) monitoring the severity and progression of photoaging and/or chronoaging; (iii) monitoring treatment efficacy; and (iv) prediction of a particular treatment or cosmetic regimen. All of these applications, which themselves represent embodiments disclosed herein, preferably use non-invasive sampling to recover information that is otherwise difficult or impractical to recover (*e.g.,* through the use of biopsies). The information may be contained in the DNA, protein, or RNA of skin cells close to the surface of the skin. In one embodiment, expression of one or more of the genes listed in Table 1, Table 2, Table 3, or any combination thereof is detected in the sample to characterize the sample. Tables 1-3 are presented by way of example to show the genes of three exemplary skin "age indexes". It should be understood that an age index can be developed by using less than the number of genes presented in any of Tables 1-3, as long as young and old stratifications are clear from the genes selected.

Other embodiments are based in part on the discovery that for tape stripping of the skin, non-polar, pliable, adhesive tapes, especially pliable tapes with rubber adhesive, are more effective than other types of adhesive tapes. Using pliable tapes with rubber adhesives, as few as 10 or less tape strippings and in certain examples as few as 4 or even 1 tape stripping can be used to isolate and/or detect nucleic acid molecules from the epidermal layer of the skin.

In another embodiment, the methods of the invention provide for determining the age range of a skin sample or of skin *in situ,* including application of a detectably labeled probe directly to the skin of a subject for visual analysis. At least one nucleic acid molecule whose expression is informative of the age range of the skin is detected on the skin using a specific probe. In one example, expression of one or more of the genes listed in Table 1, Table 2, Table 3, or any combination thereof is detected on the skin to determine the age range of the skin. In one embodiment, expression of one or more of the genes listed in Table 1, Table 2, Table 3, or any combination thereof is detected in the sample to characterize the skin sample.

In another aspect, the invention provides kits for characterizing a skin sample in a subject. In one embodiment, the kit includes a skin sample collection device, such as a biopsy needle or an adhesive tape for non-invasive tape stripping, and one or more probes or primers that selectively bind to one or more nucleic acid molecules in Table 1, Table 2, Table 3, or any combination thereof, or to a nucleic acid or protein expression product of a nucleic acid molecule in Table 1, Table 2, Table 3, or any combination thereof. The kit may include one or more pairs of forward primers that selectively bind upstream of a gene on one strand and reverse primers that selectively bind upstream of a gene on a complementary strand. In another embodiment, the kit includes a microarray containing at least a fragment of a gene or a nucleic acid or protein product of a gene identified in Table 1, Table 2, Table 3, or any combination thereof, or any combination thereof.

In another embodiment, the kit for characterizing a skin sample from a subject includes an applicator and one or more probes or primers that selectively bind to one or more nucleic acid molecules in Table 1, Table 2, Table 3, or any combination thereof, or to a nucleic acid or protein expression product of a nucleic acid molecule in Table 1, Table 2, Table 3, or any combination thereof. In one embodiment, the probes are detectably labeled for visual identification of expression of RNA.

In another aspect, the invention provides a cosmetic formulation containing agents for reducing or increasing expression of genes. In one embodiment, the agent reduce or increase expression of the genes listed in Table 1, Table 2, Table 3, or any combination thereof. In another embodiment, the cosmetic formulation is an emulsion, a cream, a lotion, a solution, an anhydrous base, a paste, a powder, a gel, or an ointment. The emulsion may be an oil-in-water emulsion or a water-in-oil emulsion. Alternatively, the formulation may be a solution, such as an aqueous solution or a hydro-alcoholic solution. In another embodiment, the cosmetic formulation is an anhydrous base, such as a lipstick or a powder. In yet another embodiment, the formulation is comprised within an anti-aging product or a moisturizing product. The cosmetic formulation may further contain one or more of estradiol; progesterone; pregnanalone; coenzyme Q10; methylsolanomethane (MSM); copper peptide (copper extract); plankton extract (phytosome); glycolic acid; kojic acid; ascorbyl palmitate; all trans retinol; azaleic acid; salicylic acid; broparoestrol; estrone; adrostenedione; androstanediols; or sunblocks.

### BRIEF DESCRIPTION OF THE DRAWINGS

Figure 1 is a hierarchial cluster analysis of the identified 100-gene classifier distinguishing skin samples of young individuals from skin samples of old individuals. The tree configuration shown at the left of the cluster analysis is representative of the genes in the order as shown in Table 1.

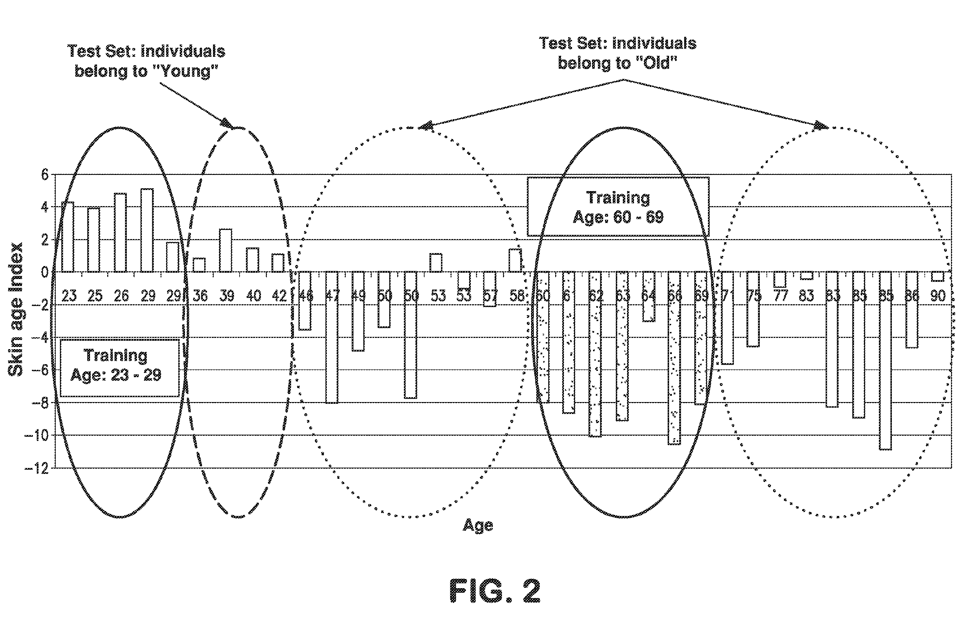

Figure 2 is a graphical diagram showing a skin age index generated from the 100-gene classifier that distinguishes skin samples of young individuals from skin samples of old individuals.

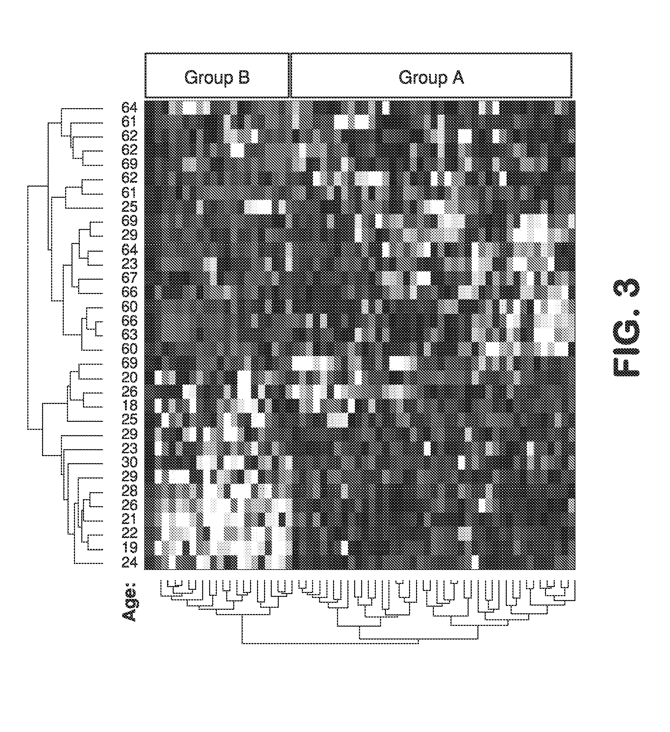

Figure 3 is a hierarchial cluster analysis of the identified 61-gene classifier distinguishing skin samples of young individuals from skin samples of old individuals. Skin age index = Sum of "Group A" - Sum of "group B" + α (constant). The tree configuration shown at the left of the cluster analysis is representative of the genes in the order as shown in Table 2.

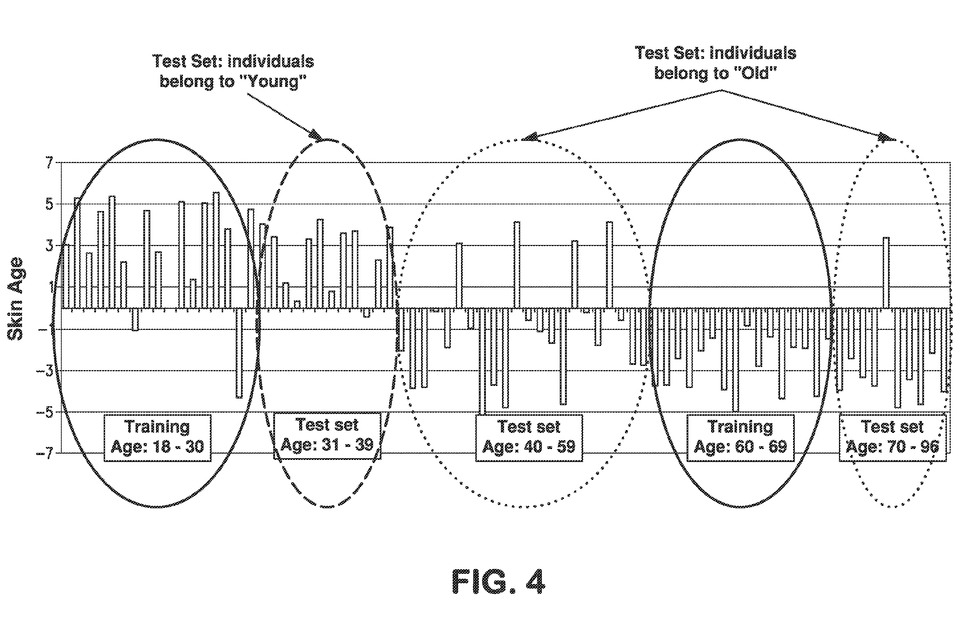

Figure 4 is a graphical diagram showing a skin age index generated from the 61-gene classifier that distinguishes skin samples of young individuals from skin samples of old individuals.

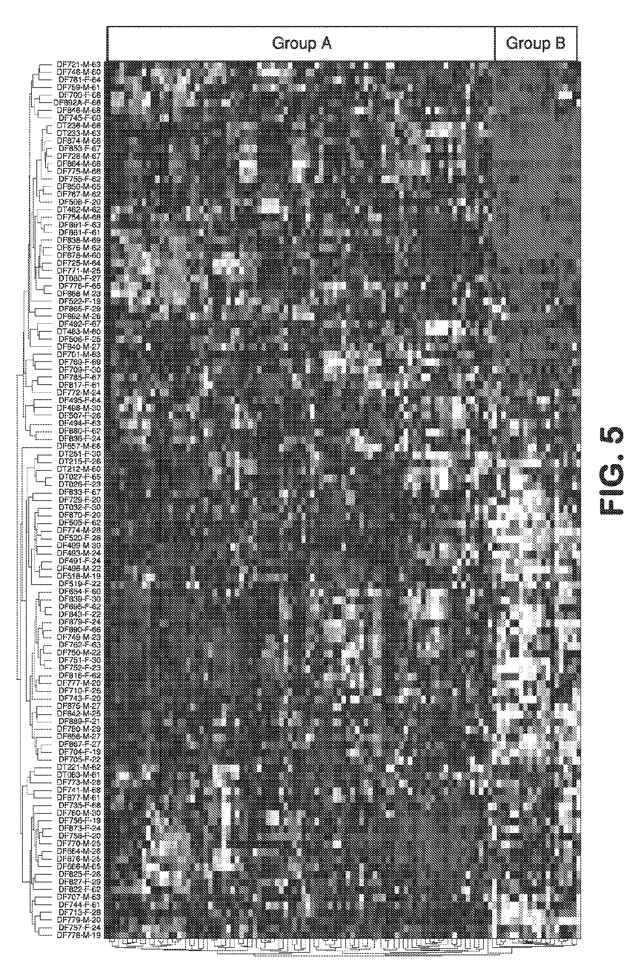

Figure 5 is a hierarchial cluster analysis of an 83-gene classifier distinguishing skin samples of young individuals from skin samples of old individuals. Skin age index = Sum of "Group A" - Sum of "group B" + α (constant). The tree configuration shown at the left of the cluster analysis is representative of the genes in the order as shown in Table 3.

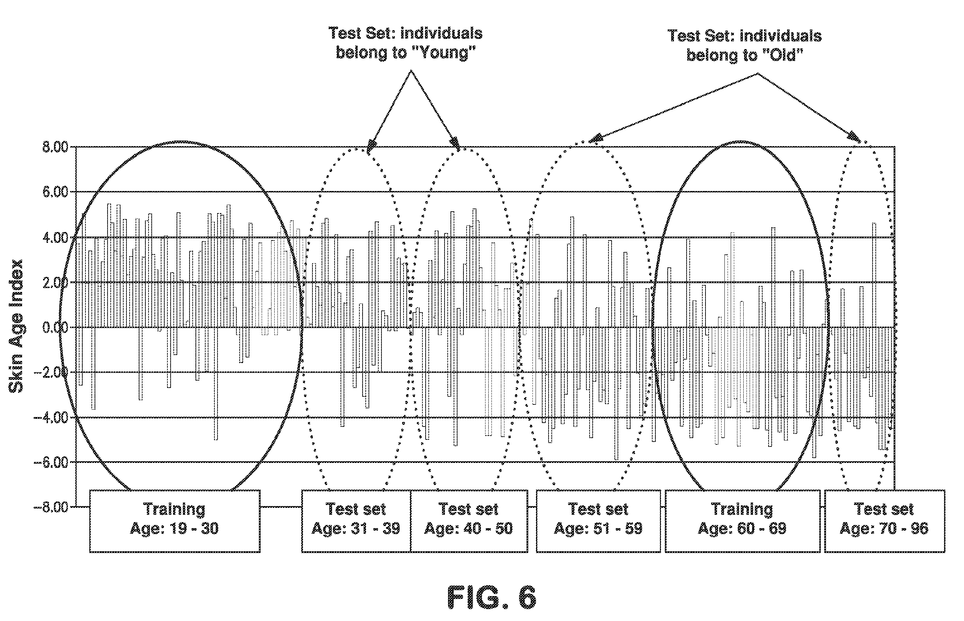

Figure 6 is a graphical diagram showing a skin age index generated from the 83-gene classifier that distinguishes skin samples of young individuals from skin samples of old individuals.

### DETAILED DESCRIPTION OF THE INVENTION

The present invention is based, in part, on the discovery that analysis of nucleic acid molecules or protein products from specific genes can be used to characterize skin samples from individuals based on an age index. Accordingly, the present invention provides methods and kits useful for characterizing a skin sample based on determining an expression profile of the sample based on identification of one or more genes or proteins.

In humans and other animals, cellular senescence has been attributed to the shortening of telomeres with each cell cycle; when telomeres become too short, the cells die. The length of telomeres is therefore the "molecular clock," predicted by Hayflick. Telomere length is maintained in immortal cells (e.g., germ cells and keratinocyte stem cells, but not other skin cell types) by the enzyme telomerase. In the laboratory, mortal cell lines can be immortalized by the activation of their telomerase gene, present in all cells but active in few cell types.

As used in this specification and the appended claims, the singular forms "a", "an", and "the" include plural references unless the context clearly dictates otherwise. Thus, for example, references to "the method" includes one or more methods, and/or steps of the type described herein which will become apparent to those persons skilled in the art upon reading this disclosure and so forth.

Unless defined otherwise, all technical and scientific terms used herein have the same meaning as commonly understood by one of ordinary skill in the art to which this invention belongs. Although any methods and materials similar or equivalent to those described herein can be used in the practice or testing of the invention, the preferred methods and materials are now described.

A number of genetic components of aging have been identified using model organisms, ranging from the simple budding yeast Saccharomyces cerevisiae to worms such as Caenorhabditis elegans and fruit flies (*Drosophila melanogaster*)*.* Study of these organisms has revealed the presence of at least two conserved aging pathways.

One of these pathways involves the gene *Sir2,* a NAD+-dependent histone deacetylase. In yeast, Sir2 is required for genomic silencing at three loci: the yeast mating loci, the telomeres and the ribosomal DNA (rDNA). In some species of yeast replicative aging may be partially caused by homologous recombination between rDNA repeats; excision of rDNA repeats results in the formation of extrachromosomal rDNA circles (ERCs). These ERCs replicate and preferentially segregate to the mother cell during cell division, and are believed to result in cellular senescence by titrating away (competing for) essential nuclear factors. ERCs have not been observed in other species of yeast (which also display replicative senescence), and ERCs are not believed to contribute to aging in higher organisms such as humans. Extrachromosomal circular DNA (eccDNA) has been found in worms, flies and humans. The role of eccDNA in aging, if any, is unknown.

Despite the lack of a connection between circular DNA and aging in higher organisms, extra copies of Sir2 are capable of extending the lifespan of both worms and flies. The mechanisms by which Sir2 homologues in higher organisms regulate lifespan is unclear, but the human SIRT1 protein has been demonstrated to deacetylate p53, Ku70, and the forkhead family of transcription factors. SIRT1 can also regulate acetylates such as CBP/p300, and has been shown to deacetylate specific histone residues.

RAS1 and RAS2 also affect aging in yeast and have a human homologue. RAS2 overexpression has been shown to extend lifespan in yeast.

Other genes regulate aging in yeast by increasing the resistance to oxidative stress. Superoxide dismutase, a protein that protects against the effects of mitochondrial free radicals, can extend yeast lifespan in stationary phase when overexpressed.

In higher organisms, aging is likely to be regulated in part through the insulin/IGF-1 pathway. Mutations that affect insulin-like signaling in worms, flies and mice are associated with extended lifespan. In yeast, Sir2 activity is regulated by the nicotinamidase PNC1. PNC1 is transcriptionally upregulated under stressful conditions such as caloric restriction, heat shock, and osmotic shock. By converting nicotinamide to niacin, it removes nicotinamide, which inhibits the activity of Sir2. A nicotinamidase found in humans, known as PBEF, may serve a similar function, and a secreted form of PBEF known as visfatin may help to regulate serum insulin levels. It is not known, however, whether these mechanisms also exist in humans since there are obvious differences in biology between humans and model organisms.

Sir2 activity has been shown to increase under calorie restriction. Due to the lack of available glucose in the cells more NAD+ is available and can activate Sir2. Resveratrol, a polyphenol found in the skin of red grapes, was reported to extend the lifespan of yeast, worms, and flies. It has been shown to activate Sir2 and therefore mimics the effects of calorie restriction.

The particularly important causes of chronological aging of human skin likely vary among a population of elderly humans, including such factors as diet, genetics, and environment. In general, though, it is believed that chronological skin aging is due to activation of the stress-activated pathways (SAPs) and a repression of the mitogen-activated pathways (ERK). However, contrary to conventional wisdom, it has been found that chronoaging and photoaging of human skin have a similar molecular pathophysiology. ERK mediates the actions of growth factors necessary for healthy skin. Interference with ERK can lead to thinning of chronologically-aged skin because of reduced number of cells in the epidermis and dermis. Almost conversely, SAPs activate factors (*e.g.*, c-Jun) that promote both inhibition of procollagen synthesis and degradation of mature collagen, and thereby lead to reduced form, strength, and function of skin. Chronological aging of skin might be expected to include some interference with ERK and/or some activation of the SAPs.

Gene expression is imperfectly controlled, and it is possible that random fluctuations in the expression levels of many genes contribute to the aging process as suggested by a study of such genes in yeast. Individual cells, which are genetically identical, none-the-less can have substantially different responses to outside stimuli, and markedly different lifespans, indicating the epigenetic factors play an important role in gene expression and aging as well as genetic factors.

Accordingly, in one embodiment, the present invention employs a non-invasive tape stripping technology to obtain samples of skin from individuals. As such, DNA microarray assays are used to create a skin age index to predict the aging of an individual. Tape-stripping removes superficial cells from the surface of the skin as well as adnexal cells. Small amounts of nucleic acid molecules isolated from tape-stripped cells can be amplified and used for microarray analyses and quantitative PCR. In addition, proteins obtained from the lysed cells may be quantitated for characterization and determination of age. Consequently, tape-stripping is a non-invasive diagnostic method, which does not interfere with subsequent histological analyses. While tape stripping will primarily sample superficial cells from the epidermis, this method holds great promise in the determination of age and age-related disorders. Consequently, this feature may help characterize an individual as having skin characterized as being younger or older than the actual age of the individual. Further, there are changes in the dermis and epidermis resulting from environmental factors, such as exposure to UV radiation. Accordingly, the present invention demonstrates that stratum corneum RNA, harvested by tape stripping with Epidermal Genetic Information Retrieval (EGIR) (see U.S. Pat. No. 6,949,338, incorporated herein by reference), can be used to distinguish skin samples of young individuals from skin samples of old individuals.

The term "subject" or "individual" as used herein refers to any individual or patient to which the subject methods are performed. Generally the subject is human, although as will be appreciated by those in the art, the subject may be an animal. Thus other animals, including mammals such as rodents (including mice, rats, hamsters and guinea pigs), cats, dogs, rabbits, farm animals including cows, horses, goats, sheep, pigs, etc., and primates (including monkeys, chimpanzees, orangutans and gorillas) are included within the definition of subject.

As used herein, the terms "sample" and "biological sample" refer to any sample suitable for the methods provided by the present invention. A sample of cells can be any sample, including, for example, a skin sample obtained by non-invasive tape stripping or biopsy of a subject, or a sample of the subject's bodily fluid. Thus, in one embodiment, the biological sample of the present invention is a tissue sample, e.g., a biopsy specimen such as samples from needle biopsy. In one embodiment, the term "sample" refers to any preparation derived from skin of a subject. For example, a sample of cells obtained using the non-invasive method described herein can be used to isolate nucleic acid molecules or proteins for the methods of the present invention.

As used herein "corresponding cells" or "corresponding sample" refers to cells or a sample from a subject that is from the same organ and of the same type as the cells being examined. In one aspect, the corresponding cells comprise a sample of cells obtained from a healthy individual that is age-matched or within an acceptable age range such that the sample is representative of a sample typically obtained from individuals within the range. Such corresponding cells can, but need not be, from an individual that is of the same sex as the individual providing the cells being examined. Thus, the term "normal sample" or "control sample" refers to any sample taken from a subject of similar species that is considered healthy and of a known age. As such, a normal/standard level of RNA denotes the level of RNA present in a sample from a subject of known age. A normal level of RNA can be established by combining skin samples or cell extracts taken from normal healthy age-matched subjects and determining the level of one or more RNAs present. In addition, a normal level of RNA also can be determined as an average value taken from a population of subjects that fall within a known age range. Accordingly, levels of RNA in subject and control samples can be compared with the standard values. Deviation between standard and subject values establishes the parameters for characterizing age and/or distinguishing samples based on age.

The term "skin" refers to the outer protective covering of the body, consisting of the epidermis (including the stratum corneum) and the underlying dermis, and is understood to include sweat and sebaceous glands, as well as hair follicle structures. Throughout the present application, the adjective "cutaneous" can be used, and should be understood to refer generally to attributes of the skin, as appropriate to the context in which they are used. The epidermis of the human skin comprises several distinct layers of skin tissue. The deepest layer is the stratum basalis layer, which consists of columnar cells. The overlying layer is the stratum spinosum, which is composed of polyhedral cells. Cells pushed up from the stratum spinosum are flattened and synthesize keratohyalin granules to form the stratum granulosum layer. As these cells move outward, they lose their nuclei, and the keratohyalin granules fuse and mingle with tonofibrils. This forms a clear layer called the stratum lucidum. The cells of the stratum lucidum are closely packed. As the cells move up from the stratum lucidum, they become compressed into many layers of opaque squamae. These cells are all flattened remnants of cells that have become completely filled with keratin and have lost all other internal structure, including nuclei. These squamae constitute the outer layer of the epidermis, the stratum corneum. At the bottom of the stratum corneum, the cells are closely compacted and adhere to each other strongly, but higher in the stratum they become loosely packed, and eventually flake away at the surface.

As used herein, "chronoaging" refers to inevitable changes that occur over time that affect the skin of a subject. In contrast, "photoaging" refers to changes to the skin of a subject over time resulting from external environmental aggressors. Exemplary external aggressors include, but are not limited to UV rays, free radicals, chemicals, and toxins. Exemplary symptoms of chronoaging and/or photoaging of skin include, but are not limited to, loss of firmness and elasticity, dryness, loss of sheen, and lines and wrinkles. As such, the terms "sun damage" and "environmental damage," when used in reference to skin are used broadly to encompass any external environmental aggressors that may prematurely age the skin of a subject.

As used herein, the term "gene" refers to a linear sequence of nucleotides along a segment of DNA that provides the coded instructions for synthesis of RNA, which, when translated into protein, leads to the expression of hereditary character. As such, the term "skin marker" or "biomarker" refers to a gene whose expression level is different between skin surface samples from individuals of distinct ages or age ranges, and skin surface samples of individuals of known age or age range. Therefore, expression of a skin marker of the invention is related to, or indicative of, the age of the subject being tested. Many statistical techniques are known in the art, which can be used to determine whether a statistically significant difference in expression is observed at a high (*e.g.,* 90% or 95%) confidence level. As such, an increase or decrease in expression of these genes is related to and can characterize age of the subject.

As used herein, the term "nucleic acid molecule" means DNA, RNA (*e.g.,* messenger RNA, miRNA, etc.), single-stranded, double-stranded or triple stranded and any chemical modifications thereof. Virtually any modification of the nucleic acid is contemplated. A "nucleic acid molecle" can be of almost any length, from 10, 20, 30, 40, 50, 60, 75, 100, 125, 150, 175, 200, 225, 250, 275, 300, 400, 500, 600, 700, 800, 900, 1000, 1500, 2000, 2500, 3000, 3500, 4000, 4500, 5000, 6000, 7000, 8000, 9000, 10,000, 15,000, 20,000, 30,000, 40,000, 50,000, 75,000, 100,000, 150,000, 200,000, 500,000, 1,000,000, 1,500,000, 2,000,000, 5,000,000 or even more bases in length, up to a full-length chromosomal DNA molecule. For methods that analyze expression of a gene, the nucleic acid isolated from a sample is typically RNA.

Micro-RNAs (miRNA) are small single stranded RNA molecules an average of 22 nucleotides long that are involved in regulating mRNA expression in diverse species including humans. Hundreds of miRNAs have been discovered in flies, plants and mammals. miRNAs regulate gene expression by binding to the 3'-untranslated regions of mRNA and catalyze either i) cleavage of the mRNA; or 2) repression of translation. The regulation of gene expression by miRNAs is central to many biological processes such as cell development, differentiation, communication, and apoptosis. Recently it has been shown that miRNA are active during embryogenesis of the mouse epithelium and play a significant role in skin morphogenesis.

Given the role of miRNA in gene expression it is clear that miRNAs will influence, if not completely specify the relative amounts of mRNA in particular cell types and thus determine a particular gene expression profile (*i.e.,* a population of specific mRNAs) in different cell types. In addition, it is likely that the particular distribution of specific miRNAs in a cell will also be distinctive in different cell types. Thus, determination of the miRNA profile of a tissue may be used as a tool for expression profiling of the actual mRNA population in that tissue. Accordingly, miRNA levels are useful for the purposes of characterization of a subject within an age range.

As used herein, the term "protein" refers to at least two covalently attached amino acids, which includes proteins, polypeptides, oligopeptides and peptides. A protein may be made up of naturally occurring amino acids and peptide bonds, or synthetic peptidomimetic structures. Thus "amino acid", or "peptide residue", as used herein means both naturally occurring and synthetic amino acids. For example, homo-phenylalanine, citrulline and noreleucine are considered amino acids for the purposes of the invention. "Amino acid" also includes imino acid residues such as proline and hydroxyproline. The side chains may be in either the (R) or the (S) configuration.

A "probe" or "probe nucleic acid molecule" is a nucleic acid molecule that is at least partially single-stranded, and that is at least partially complementary, or at least partially substantially complementary, to a sequence of interest. A probe can be RNA, DNA, or a combination of both RNA and DNA. It is also within the scope of the present invention to have probe nucleic acid molecules comprising nucleic acids in which the backbone sugar is other that ribose or deoxyribose. Probe nucleic acids can also be peptide nucleic acids. A probe can comprise nucleolytic-activity resistant linkages or detectable labels, and can be operably linked to other moieties, for example a peptide.

A single-stranded nucleic acid molecule is "complementary" to another single-stranded nucleic acid molecule when it can base-pair (hybridize) with all or a portion of the other nucleic acid molecule to form a double helix (double-stranded nucleic acid molecule), based on the ability of guanine (G) to base pair with cytosine (C) and adenine (A) to base pair with thymine (T) or uridine (U). For example, the nucleotide sequence 5'-ATAC-3' is complementary to the nucleotide sequence 5'-GTAT-3'.

The term "antibody" as used in this invention is meant to include intact molecules of polyclonal or monoclonal antibodies, as well as fragments thereof, such as Fab and F(ab')₂, Fv and SCA fragments which are capable of binding an epitopic determinant. The term "specifically binds" or "specifically interacts," when used in reference to an antibody means that an interaction of the antibody and a particular epitope has a dissociation constant of at least about 1 x 10⁻⁶, generally at least about 1 x 10⁻⁷, usually at least about 1 x 10⁻⁸, and particularly at least about 1 x 10⁻⁹ or 1 x 10⁻¹⁰ or less.

As used herein "hybridization" refers to the process by which a nucleic acid strand joins with a complementary strand through base pairing. Hybridization reactions can be sensitive and selective so that a particular sequence of interest can be identified even in samples in which it is present at low concentrations. In an *in vitro* situation, suitably stringent conditions can be defined by, for example, the concentrations of salt or formamide in the prehybridization and hybridization solutions, or by the hybridization temperature, and are well known in the art. In particular, stringency can be increased by reducing the concentration of salt, increasing the concentration of formamide, or raising the hybridization temperature. For example, hybridization under high stringency conditions could occur in about 50% formamide at about 37°C to 42°C. Hybridization could occur under reduced stringency conditions in about 35% to 25% formamide at about 30°C to 35°C. In particular, hybridization could occur under high stringency conditions at 42°C in 50% formamide, 5X SSPE, 0.3% SDS, and 200 mg/ml sheared and denatured salmon sperm DNA. Hybridization could occur under reduced stringency conditions as described above, but in 35% formamide at a reduced temperature of 35°C. The temperature range corresponding to a particular level of stringency can be further narrowed by calculating the purine to pyrimidine ratio of the nucleic acid of interest and adjusting the temperature accordingly. Variations on the above ranges and conditions are well known in the art.

As used herein, the term "mutation" refers to a change in the genome with respect to the standard wild-type sequence. Mutations can be deletions, insertions, or rearrangements of nucleic acid sequences at a position in the genome, or they can be single base changes at a position in the genome, referred to as "point mutations." Mutations can be inherited, or they can occur in one or more cells during the lifespan of an individual.

As used herein, the term "kit" or "research kit" refers to a collection of products that are used to perform a biological research reaction, procedure, or synthesis, such as, for example, a detection, assay, separation, purification, etc., which are typically shipped together, usually within a common packaging, to an end user.

Samples from a tissue can be isolated by any number of means well known in the art. Invasive methods for isolating a sample include, but are not limited to the use of needles or scalpels, for example during biopsies of various tissues. Non-invasive methods for isolating a sample include, but are not limited to tape-stripping and skin scraping.

As such, the tape stripping methods provided herein typically involve applying an adhesive tape to the skin of a subject and removing the adhesive tape from the skin of the subject one or more times. In certain examples, the adhesive tape is applied to the skin and removed from the skin about one to ten times. Alternatively, about ten adhesive tapes can be sequentially applied to the skin and removed from the skin. These adhesive tapes are then combined for further analysis. Accordingly, an adhesive tape can be applied to and removed from a target site 10, 9, 8, 7, 6, 5, 4, 3, 2, or 1 time, and/or 10, 9, 8, 7, 6, 5, 4, 3, 2, or 1 adhesive tape can be applied to and removed from the target site. In one illustrative example, the adhesive tape is applied to the skin between about one and eight times, in another example, between one and five times, and in another illustrative example the tape is applied and removed from the skin four times.

The rubber based adhesive can be, for example, a synthetic rubber-based adhesive. The rubber based adhesive in illustrative examples, has high peel, high shear, and high tack. For example, the rubber based adhesive can have a peak force tack that is at least 25%, 50%, or 100% greater than the peak force tack of an acrylic-based tape such as D-SQUAME™. D-SQUAME ™ has been found to have a peak force of 2 Newtons, wherein peak force of the rubber based adhesive used for methods provided herein, can be 4 Newtons or greater. Furthermore, the rubber based adhesive can have adhesion that is greater than 2 times, 5 times, or 10 times that of acrylic based tape. For example, D-SQUAME ™ has been found to have adhesion of 0.0006 Newton meters, whereas the rubber based tape provided herein can have an adhesion of about 0.01 Newton meters using a texture analyzer. Furthermore, in certain illustrative examples, the adhesive used in the methods provided herein has higher peel, shear and tack than other rubber adhesives, especially those used for medical application and Duct tape.

Virtually any size and/or shape of adhesive tape and target skin site size and shape can be used and analyzed, respectively, by the methods of the present invention. For example, adhesive tape can be fabricated into circular discs of diameter between 10 millimeters and 100 millimeters, for example between 15 and 25 millimeters in diameter. The adhesive tape can have a surface area of between about 50 mm² and 1000 mm², between about 100 mm² to 500 mm² or about 250 mm².

In another embodiment, the sample may be obtained by means of an invasive procedure, such as biopsy. Biopsies may be taken instead of or after tape stripping and are subjected to standard histopathologic analysis. Analysis of biopsy samples taken simultaneously with tape stripping samples may then be correlated with the data generated from one or more of analysis of selected lesion RNA samples by DNA microarray, correlation of gene expression data with histopathology, and creation of a candidate expression classifier for the skin age index.

As used herein, "biopsy" refers to the removal of cells or tissues for analysis. There are many different types of biopsy procedures known in the art. The most common types include: (1) incisional biopsy, in which only a sample of tissue is removed; (2) excisional biopsy, in which an entire lump or suspicious area is removed; and (3) needle biopsy, in which a sample of tissue or fluid is removed with a needle. When a wide needle is used, the procedure is called a core biopsy. When a thin needle is used, the procedure is called a fine-needle aspiration biopsy. Other types of biopsy procedures include, but are not limited to, shave biopsy, punch biopsy, curettage biopsy, and *in situ* biopsy. In another embodiment, the skin sample is obtained by scraping the skin with an instrument to remove one or more nucleic acid molecules from the skin.

The skin sample obtained using the tape stripping method includes, epidermal cells including cells comprising adnexal structures. In certain illustrative examples, the sample includes predominantly epidermal cells, or even exclusively epidermal cells. The epidermis consists predominantly of keratinocytes (> 90%), which differentiate from the basal layer, moving outward through various layers having decreasing levels of cellular organization, to become the cornified cells of the stratum corneum layer. Renewal of the epidermis occurs every 20-30 days in uninvolved skin. Other cell types present in the epidermis include melanocytes, Langerhans cells, and Merkel cells. As illustrated in the Examples herein, the tape stripping method of the present invention is particularly effective at isolating epidermal samples.

Nucleic acid molecules can also be isolated by lysing the cells and cellular material collected from the skin sample by any number of means well known to those skilled in the art. For example, a number of commercial products available for isolating polynucleotides, including but not limited to, RNeasy™ (Qiagen, Valencia, CA) and TriReagent™ (Molecular Research Center, Inc, Cincinnati, OH) can be used. The isolated polynucleotides can then be tested or assayed for particular nucleic acid sequences, including a polynucleotide encoding a cytokine. Methods of recovering a target nucleic acid molecule within a nucleic acid sample are well known in the art, and can include microarray analysis.

Nucleic acid molecules may be analyzed in any number of ways known in the art. For example, the presence of nucleic acid molecules can be detected by DNA-DNA or DNA-RNA hybridization or amplification using probes or fragments of the specific nucleic acid molecule. Nucleic acid amplification based assays involve the use of oligonucleotides or oligomers based on the nucleic acid sequences to detect transformants containing the specific DNA or RNA.

In one embodiment, analysis of the nucleic acid molecules includes genetic analysis to determine the nucleotide sequence of a gene. Since a difference in length or sequence between DNA fragments isolated from a sample and those of known sequences are due to an insertion, deletion, or substitution of one or more nucleotides, the determination of nucleic acid sequences provides information concerning mutations resulting from environmental affects on the skin of individuals. These mutations may also include transposition or inversion and are difficult to detect by techniques other than direct sequencing. Accordingly, the methods of the present invention may be used to detect genetic mutations in one or more genes listed in Table 1, Table 2, Table 3, or any combination thereof for determination and/or characterization of the age of the subject.

A variety of protocols for detecting and measuring the expression of nucleic acid molecules, using either polyclonal or monoclonal antibodies specific for the protein expression product are known in the art. Examples include enzyme-linked immunosorbent assay (ELISA), radioimmunoassay (RIA), and fluorescence activated cell sorting (FACS). These and other assays are described, among other places, in Hampton, R. et al. (1990; Serological Methods, a Laboratory Manual, APS Press, St Paul, Minn.) and Maddox, D. E. et al. (1983; J. Exp. Med. 158:1211-1216).

In another embodiment, antibodies that specifically bind to the expression products of the nucleic acid molecules of the invention may be used to characterize the skin sample of the subject. The antibodies may be used with or without modification, and may be labeled by joining them, either covalently or non-covalently, with a reporter molecule.

A wide variety of labels and conjugation techniques are known by those skilled in the art and may be used in various nucleic acid and amino acid assays. Means for producing labeled hybridization or PCR probes for detecting sequences related to the nucleic acid molecules of Table 1, Table 2, Table 3, or any combination thereof include oligolabeling, nick translation, end-labeling or PCR amplification using a labeled nucleotide. Alternatively, the nucleic acid molecules, or any fragments thereof, may be cloned into a vector for the production of an mRNA probe. Such vectors are known in the art, are commercially available, and may be used to synthesize RNA probes *in vitro* by addition of an appropriate RNA polymerase such as T7, T3, or SP6 and labeled nucleotides. These procedures may be conducted using a variety of commercially available kits (Pharmacia & Upjohn, (Kalamazoo, Mich.); Promega (Madison Wis.); and U.S. Biochemical Corp., Cleveland, Ohio). Suitable reporter molecules or labels, which may be used for ease of detection, include radionuclides, enzymes, fluorescent, chemiluminescent, or chromogenic agents as well as substrates, cofactors, inhibitors, magnetic particles, and the like.

PCR systems usually use two amplification primers and an additional amplicon-specific, fluorogenic hybridization probe that specifically binds to a site within the amplicon. The probe can include one or more fluorescence label moieties. For example, the probe can be labeled with two fluorescent dyes: 1) a 6-carboxy-fluorescein (FAM), located at the 5'-end, which serves as reporter, and 2) a 6-carboxy-tetramethyl-rhodamine (TAMRA), located at the 3'-end, which serves as a quencher. When amplification occurs, the 5'-3' exonuclease activity of the Taq DNA polymerase cleaves the reporter from the probe during the extension phase, thus releasing it from the quencher. The resulting increase in fluorescence emission of the reporter dye is monitored during the PCR process and represents the number of DNA fragments generated. *In situ* PCR may be utilized for the direct localization and visualization of target nucleic acid molecules and may be further useful in correlating expression with chronoaging or photoaging of skin.

Means for producing specific hybridization probes for nucleic acid molecules of the invention include the cloning of the nucleic acid sequences into vectors for the production of mRNA probes. Such vectors are known in the art, commercially available, and may be used to synthesize RNA probes *in vitro* by means of the addition of the appropriate RNA polymerases and the appropriate labeled nucleotides. Hybridization probes may be labeled by a variety of reporter groups, for example, radionuclides such as 32P or 35S, or enzymatic labels, such as alkaline phosphatase coupled to the probe via avidin/biotin coupling systems, and the like.

In order to provide a basis for the determination or characterization of chronoaging and/or photoaging of skin associated with expression of the nucleic acid molecules of the invention, a normal or standard profile for expression is established. Such a standard profile may be used to develop a skin age index for comparison to test samples of individuals of unknown age. Standard hybridization may be quantified by comparing the values obtained from subjects of known skin characterization or age range (e.g., from subjects falling with the range of "young" (*i.e.,* ages of about 18-30, about 19-30, about 23-29 or about 23-42) or subjects falling within the range of "old" (*i.e.,* ages of about 60-69 or about 46-90)). Standard values obtained from such samples may be compared with values obtained from samples from subjects of known age and/or known age range. Deviation between standard and subject values is used to characterize the skin of a subject.

Accordingly, in one aspect of the invention, a non-invasive sampling method is provided for the characterization of the skin of a subject. In one embodiment, a sample set of skin samples of individuals of known age is created. Each sample consists of nucleic acid molecules recovered by tape stripping or biopsy sample of the superficial epidermis of the individuals of known age range. In addition to tape striping, a standard biopsy of the same lesion may also be performed, along with accompanying analysis and characterization. Nucleic acid molecules recovered by tape stripping the superficial epidermis of normal skin will serve as a negative control.

In another aspect, the invention provides a method of distinguishing young individuals from old individuals. In one embodiment, the method includes analyzing a nucleic acid molecule from one or more genes listed in Table 1, Table 2, Table 3, or any combination thereof. The skin sample of a subject of unknown age range is assayed for expression of a large number of genes. Analyzing expression includes any qualitative or quantitative method for detecting expression of a gene, many of which are known in the art. The method can include analyzing expression of specific markers by measuring expression of the markers using a quantitative method, or by using a qualitative method. Non-limiting methods for analyzing polynucleotides and polypeptides are discussed below.

Methods of analyzing expression of a gene of the present invention can utilize a microarray, or other miniature high-throughput technology, for detecting expression of one or more gene products. Quantitative measurement of expression levels using such microarrays is also known in the art, and typically involves a modified version of a traditional method for measuring expression as described herein. For example, such quantitation can be performed by measuring a phosphor image of a radioactive-labeled probe binding to a spot of a microarray, using a phospohor imager and imaging software.

By identifying gene sets that are unique to a given age range, differences in the genetic expression can be utilized for characterization of individuals of unknown age. In one embodiment, the nucleic acid molecule is RNA, including messenger RNA (mRNA) that is isolated from a sample from the subject. Up-regulated and down-regulated gene sets for a given disease state may be subsequently combined. The combination enables those of skill in the art to identify gene sets or panels that are unique to a given age range. Such gene sets are of immense determinative and characteristic value as they can be routinely used in assays that are simpler than microarray analysis (for example "real-time" quantitative PCR). Such gene sets also provide insights into pathogenesis and targets for the design of new drugs.

A reference database containing a number of reference projected profiles is also created from skin samples of subjects of known age and/or age range, such as, for example, "young" (*i.e.,* ages of about 18-30, about 19-30, about 23-29 or about 23-42) or "old" (*i.e.,* ages of about 60-69 or about 46-90). The projected profile is then compared with the reference database containing the reference projected profiles. If the projected profile of the subject matches best with the profile of a particular age range in the database, the subject is determined to have skin characteristic of an individual within the identified age range. Various computer systems and software can be utilized for implementing the analytical methods of this invention and are apparent to one of skill in the art. Exemplary software programs include, but are not limited to, Cluster & TreeView (Stanford, URLs: rana.1b1.gov or microarray.org), GeneCluster (MIT/Whitehead Institute, URL: MPR/GeneCluster/GeneCluster.html), Array Explorer (SpotFire Inc, URL: spotfire.com/products/scicomp.asp#SAE) and GeneSpring (Silicon Genetics Inc, URL: sigenetics.com/Products/GeneSpring/index.html) (for computer systems and software, see also U.S. Pat. No. 6,203,987, incorporated herein by reference).

In another aspect, the methods of the present invention involve *in situ* analysis of the skin for characterization thereof. For *in situ* methods, nucleic acid molecules do not need to be isolated from the subject prior to analysis. In one embodiment, detectably labeled probes are contacted with a cell or tissue of a subject for visual detection of expressed RNA to characterize the skin as discussed above.

In another aspect, the methods of the present invention can also be useful for monitoring the progression of chronoaging and/or photoaging, and for monitoring the effectiveness of one or more treatments for the symptoms of chronoaging and/or photoaging. For example, by comparing the projected profile prior to treatment with the profile after treatment.

In a related aspect, the methods of the present invention can also be useful for determining an appropriate treatment regimen for a subject having a specific symptom of chronoaging and/or photoaging. Thus, the methods of the invention are useful for providing a means for practicing personalized medicine, wherein treatment is tailored to a subject based on the particular characteristics of the skin of the subject. The method can be practiced, for example, by first characterizing the skin of the subject, as described above.

Once photoaging and/or chronoaging of the skin of a subject is established and a treatment protocol is initiated, the methods of the invention may be repeated on a regular basis to monitor the expression profiles of the genes of interest in the subject. The results obtained from successive assays may be used to show the efficacy of treatment over a period ranging from several days to months. Accordingly, another aspect of the invention is directed to methods for monitoring a therapeutic regimen for treating a subject having symptoms of photoaging and/or chronoaging. A comparison of the expression profile or mutations in the nucleic acid sequence of the nucleic acid molecule prior to and during therapy will be indicative of the efficacy of the therapy. Therefore, one skilled in the art will be able to recognize and adjust the therapeutic approach as needed.

The efficacy of a therapeutic regimen for treating symptoms of photoaging and/or chronoaging can be identified by an absence of symptoms or clinical signs characteristic of the age range of the subject at the time of onset of therapy. For example, restoration of skin elasticity, reduction of wrinkles, and/or restoration of skin density may all be used to identify efficacy of a therapeutic regimen.

When performed in a high throughput (or ultra-high throughput) format, the methods of the invention can be performed on a solid support (*e.g*., a microtiter plate, a silicon wafer, or a glass slide), wherein cell samples and/or genes of interest are positioned such that each is delineated from each other (*e.g.*, in wells). Any number of samples or genes (*e.g.,* 96, 1024, 10,000, 100,000, or more) can be examined in parallel using such a method, depending on the particular support used. Where samples are positioned in an array (*i.e.,* a defined pattern), each sample in the array can be defined by its position (*e.g.*, using an x-y axis), thus providing an "address" for each sample. An advantage of using an addressable array format is that the method can be automated, in whole or in part, such that cell samples, reagents, genes of interest, and the like, can be dispensed to (or removed from) specified positions at desired times, and samples (or aliquots) can be monitored, for example, for expression products and/or mutations in the nucleic acid sequence of the nucleic acid molecules from any one or more of the genes listed in Table 1, Table 2, Table 3, or any combination thereof.

Thus, the microarray can be used to monitor the expression level of large numbers of genes simultaneously (to produce a transcript image), and to identify genetic variants, mutations and polymorphisms. Polynucleotides used in the microarray may be oligonucleotides that are specific to a gene or genes of interest in which at least a fragment of the sequence is known or that are specific to one or more unidentified cDNAs which are common to a particular cell type or age range. In order to produce oligonucleotides to a known sequence for a microarray, the gene of interest is examined using a computer algorithm which starts at the 5' or more preferably at the 3' end of the nucleotide sequence. The algorithm identifies oligomers of defined length that are unique to the gene, have a GC content within a range suitable for hybridization, and lack predicted secondary structure that may interfere with hybridization. In certain situations it may be appropriate to use pairs of oligonucleotides on a microarray. The "pairs" will be identical, except for one nucleotide which preferably is located in the center of the sequence. The second oligonucleotide in the pair (mismatched by one) serves as a control. The number of oligonucleotide pairs may range from two to one million. The oligomers are synthesized at designated areas on a substrate using a light-directed chemical process. The substrate may be paper, nylon or other type of membrane, filter, chip, glass slide or any other suitable solid support.

According to another aspect of the present invention, a kit is provided that is useful for characterizing the skin of an individual, *e.g.*, using the methods provided by the present invention to determine the age range characteristic of the skin of a subject. In one embodiment, a kit of the invention includes a skin sample collection device and one or more probes or primers that selectively bind to one or more of the nucleic acid molecules of Table 1, Table 2, Table 3, or any combination thereof. In another embodiment, the kit includes one or more applicators in addition to or instead of the skin sample collection device. Such applicators are useful for *in situ* analysis of gene expression on the skin of a subject. For example, an applicator may be used to apply detectably labeled probes for visual detection of expressed RNA to characterize the skin lesion.

In another embodiment, a kit of the invention includes a probe that binds to a portion of a nucleic acid molecule in Table 1, Table 2, Table 3, or any combination thereof. In another embodiment, the kit further includes a microarray that contains at least a fragment of a gene or a nucleic acid molecule or a protein product of any one of the genes listed in Table 1, Table 2, Table 3, or any combination thereof. In some embodiments, many reagents may be provided in a kit of the invention, only some of which should be used together in a particular reaction or procedure. For example, multiple primers may be provided, only two of which are needed for a particular application.

In another embodiment, the kit of the invention provides a compartmentalized carrier including a first container containing a pair of primers. The primers are typically a forward primer that selectively binds upstream of a gene on one strand, and a reverse primer that selectively binds upstream of a gene on a complementary strand. Optionally the kits of the present invention can further include an instruction insert, *e.g.*, disclosing methods for sample collection using the sample collection device and/or exemplary gene expression profiles for comparison with the expression profile of the sample taken from the subject.

The following examples are provided to further illustrate the advantages and features of the present invention, but are not intended to limit the scope of the invention. While they are typical of those that might be used, other procedures, methodologies, or techniques known to those skilled in the art may alternatively be used.

### EXAMPLE 1

### RNA Quantitation and Profiling

This study is divided into two separate phases, a sample collection and characterization phase (phase 1) and an RNA profiling phase (phase 2). In phase 1 the tape stripped specimens and biopsied sample collections were performed by the principal investigator or trained individuals delegated by the principal investigator to obtain the biopsy sample at various sites. The RNA profiling phase (Phase 2), includes, but is not limited to RNA purification and hybridization to DNA microarrays for gene expression profiling.

**Materials and reagents.** Adhesive tape was purchased from Adhesives Research (Glen Rock, PA) in bulk rolls. These rolls were custom fabricated into small circular discs, 17 millimeters in diameter, by Diagnostic Laminations Engineering (Oceanside, CA). Human spleen total RNA was purchased from Ambion (catalogue # 7970; Austin, TX). RNeasy RNA extraction kit was purchased from Qiagen (Valencia, CA). Reverse transcriptase, PCR primers and probes, and TaqMan Universal Master Mix, which included all buffers and enzymes necessary for the amplification and fluorescent detection of specific cDNAs, were purchased from Applied Biosystems (Foster City, CA). MELT total nucleic acid isolation system was purchased from Ambion (Austin, TX).

**RNA isolation.** RNA was extracted from tapes using either pressure cycling technology (PCT; Garrett, Tao et al. 2002; Schumacher, Manak et al. 2002) or MELT total nucleic acid system. Tapes were extracted in pairs by insertion into a PULSE™ tube (Pressure Biosciences, Gaithersburg, MD) with 1.2 mls of buffer RLT (supplied in the Qiagen RNeasy kit). PULSE™ tubes were inserted into the PCT-NEP2017 pressure cycler and the sample was extracted using the following parameters: room temperature; 5 pressure cycles of 35 Kpsi with pressure held for 20 seconds at the top and bottom of each cycle. After pressure extraction the buffer was removed and used to process the remaining tapes used to strip that site; the buffer was then processed according to the standard Qiagen RNeasy protocol for the collection of larger RNAs (>200 nucleotides) by application to a purification column to which large RNA molecules (*i.e.* mRNAs) bind, while the column flow-through is saved for microRNA purification. The column flow-through, which contains miRNA separated from mRNA, is processed according to the Qiagen miRNA purification procedure (on the world wide web at qiagen.com/literature/protocols/pdf/RY20.pdf) to purify the microRNA. RNA from the 2 sites stripped on each subject was pooled to create a single sample from each subject.

**RNA isolation using MELT total nucleic acid protocol.** Tapes were extracted in a 2 ml eppendorf tube with 192 µl MELT buffer plus 8 µl of MELT cocktail and vortexed for 10 minutes at room temperature. The MELT lysates were transferred to the dispensed binding bead master mix after spinning down for 3 minutes at >10,000 xg and washed with 300 µl of Wash Solution 1 and 2. RNAs were eluted in 100 µl of elution solution.

**Quantitation of mRNA.** Experimental data is reported as the number of PCR cycles required to achieve a threshold fluorescence for a specific cDNA and is described as the "Cₜ" value (Gibson, Heid et al. 1996; Heid, Stevens et al. 1996; AppliedBiosystems 2001). Quantitation of total RNA mass was performed as previously described (Wong, Tran et al. 2004). Briefly, RNA mass recovered from tapes is determined by using quantitative RT-PCR with reference to a standard curve (C_{t, actin} vs. log[RNA]; AppliedBiosystems 2001) created from commercially purchased human spleen total RNA. The average of 6 replicate C_{t, actin} values was used to calculate the concentration of RNA in a sample with reference to the standard curve.

**RNA amplification and array hybridization.** RNA was isolated by the Multi-Enzymatic Liquefaction of Tissue method (Ambion, Austin, TX) and amplified using the WT-Ovation pico amplification system (NuGen, San Carlos, CA). The amplified RNA was hybridized to Affymetrix U133 plus 2.0 microarray and data were processed and analyzed using R from Bioconductor.

**Preprocessing GeneChip Data.** The image files from scanning the Affymetrix GeneChips with the Affymetrix series 3000 scanner will be converted using GCOS software (Affymetrix) to "CEL" format files. Normalization of CEL files will be carried out using software from the Bioconductor suite (on the world wide web at bioconductor.org). In particular, a robust multiarray analysis with adjustments for optical noise and binding affinities of oligonucleotide probes (Wu et al., 2006; and Wu et al., 2004) as implemented by the function "just.gcrma" in the "gcrma" package will be used to normalize the GeneChip Data.

**Statistical Approach for Microarray Data Analysis.** Two generic statistical problems are addressed in this proposal: (i) identifying genes that are differentially expressed in different age ranges (*i.e.* young versus old) and (ii) forming (and evaluating) rules for classification of young and old skin samples into groups based on gene expression data.

The methods that will be used to address the problems identified above are now standard in the statistical evaluation of microarray data. These methods have been applied by others to data from Affymetrix arrays to study gene expression in prostate cancer, to characterize changes in gene expression subsequent to HIV infection, and to develop a high throughput genotyping platform. For identifying differentially expressed genes, permutation based estimates of false discovery rates are preferred. Scripts for the R quantitative programming environment were developed to implement these methods in our previous work, but will likely use or adapt the "siggenes" package from the Bioconductor suite in this project. The development of classification rules will rely on resampling methods (k-fold cross-validation, the 632 plus bootstrap, and/or bagging applied to the naive Bayes classifier and the nearest shrunken centroid classifier and the support vector machine (SVM) which both performed well in classifying prostate tissues as malignant or benign, used in our previous work. The implementation likely to be used is to perform k-fold cross-validation. Within each of the k train/test cycles an initial screen of the training data for differentially expressed genes is performed and genes are ordered according to their posterior probability of differential expression. Naive Bayes and nearest shrunken centroid classifiers based on the r genes with the highest posterior probability of differential expression are formed choosing enough values of r between 1 and 1024 to allow accurate interpolation of the classification error rate. The "one se rule" is applied to the error rates for the test sets to choose the classifier that minimizes the error rate. For SVM, an internal 632+ bootstrap is applied to each training sample to select the number of genes to be used in forming the classifier. The "1 se rule" error rates from the k test sets are used to characterize the classification accuracy.

In addition to the use of univariate and multivariate statistical analysis tools, sophisticated bioinformatic analysis approaches will help make sense of possible biological links between the genes found to be differentially expressed between, *e.g.*, normal aging and advanced aging samples. These approaches will focus on the analysis of genetic networks and pathways and have been implemented in software packages such as Ingenuity (on the world wide web at ingenuity.com) and MetaCore (on the world wide web at genego.com). The identification of the biological links between genes that emerge from a gene expression microarray analysis can help put into context the biological meaningfulness of their expression patterns as well as help reduce the set of differentially expressed genes to be represented on a diagnostic panel based on their biology. The end result of this analysis will be to define a candidate expression classifier that will be validated in future, larger clinical trials.

**QC metrics for RNA, amplified cDNA and microarray data.** Following informed consent, the skin of subjects was tape stripped using EGIR. The resulting RNA isolated from the EGIR tape was amplified and profiled on the Affymetrix U133 plus 2.0 GeneChip. Microarray data were normalized by the GCRMA algorithm. To assure high quality of microarray data are generated, QC metrics were established for RNA, amplified cDNA and microarray data. The quality of RNA was assessed by capillary electrophoresis using the Experion system (Biorad, Hercule, CA) and RNA with at least one visible 18S rRNA was further processed for RNA amplification. The amplified cDNA was quantified by the Nanodrop system and quality of the amplified cDNA was also assessed by the Experion system. The yield of the amplified cDNAs greater than 5 µg and the average size distribution of the cDNAs greater than 750 nt were carried forward for microarray hybridization. Quality of the array data was further assessed using simpleaffy program in R and the array data with scaling factor less than 5.0 and % present call greater than 30% were used for further data analysis.

**Class Modeling - PAM.** After passing the array data QC, skin specimens from "young" and "old" individuals were further analyzed and three separate gene classifiers identified.

First, gene expression values less than 100 across all samples were filtered out and 14000 probesets were tested. These 14000 probesets were subjected to a statistical analysis for differentially expressed genes among "young" and "old" skin using ANOVA (p<0.05), multiple testing correction algorithm (Westafall and Young permutation) and false discover rate (FDR) of 0.05. With a false discovery rate of q < 0.05, the results showed a two-fold difference between "young" and "old" samples, and identified 483 differentially expressed genes. A 100-gene panel (Table 1) was found to be a potential classifer that discerned skin from "young" and "old" individuals. The genes and respective classifier panels were analyzed using the Prediction Analysis of Microarrays (PAM) software freely available from Stanford University (Stanford, CA).

Second, approximately 14,000 genes were subjected to statistical analysis for differentially expressed genes after eliminating very low expressed genes (expression level < 100 across all samples). t-test was performed to compare "young" (18 ∼ 30) and "old" (60 ∼ 69), (p<0.05). Multiple testing correction using Benjamini and Hochberg methods were performed. With a false discovery rate of q < 0.05, the results showed a two-fold difference between "young" and "old" samples, and identified 313 differentially expressed genes. PAM was used to rank these genes: between "young" and "old" and a 61-gene classifier was selected (Table 2).

Third, approximately 24,200 genes were subjected to statistical analysis for differentially expressed genes after eliminating very low expressed genes (expression level < 100 across all samples). t-test was performed to compare "young" and "old" samples (p<0.01), which were divided into four groups: age 31 - 39, 40 - 50 , 51 - 59 and 70 - 96. Age ranges, 31 - 39 and 40 - 50, belong to the "Young" group, while age ranges, 51 - 59 and 70 - 96, belong to the "Old" group. Multiple testing correction using Benjamini and Hochberg methods were performed. With a false discovery rate of q < 0.05, the results identified 2354 differentially expressed genes. PAM was used to rank these genes: between "young" and "old" and an 83 gene(106 probesets) classifier was selected (Table 3).

The PAM software uses a modification of the nearest centroid method, which computes a standardized centroid for each class in a training set. This refers to the average gene expression for each gene in each class divided by the within-class standard deviation for that gene. Nearest centroid classification takes the gene expression profile of a new sample, and compares it to each of these class centroids. The class, whose centroid it is closest to, in squared distance, is the predicted class for that new sample.

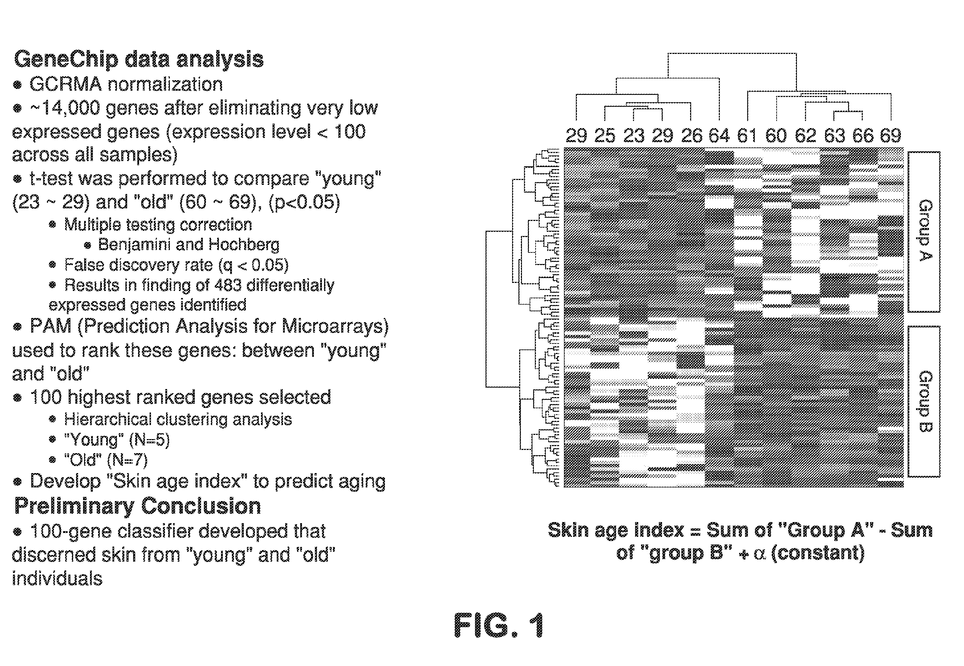

These genes were all subjected to a hierarchical clustering analysis (Figure 1), with the "young" specimens grouped together and clearly distinguished from "old" specimens. These data suggest stratum corneum RNA, harvested by tape stripping with EGIR, can be used to distinguish and/or characterize skin as being "young" or "old." Thus, RNA harvested by EGIR technology is more than adequate for microarray-based gene expression profiling and appropriately reflects the pathologic state of skin.

### EXAMPLE 2

### Tape Stripping to Recover Nucleic Acids from Skin

The following procedure was used to recover nucleic acids from normal skin (e.g., the mastoid or upper back areas) of a subject.

Tapes were handled with gloved hands at all times. A particular site that is relatively blemish-free and healthy was located, unless otherwise specified by the protocol. Preferred normal skin sites are the mastoid process (the bony process behind the ear at the base of the skull) and the upper back, immediately superior to the scapular spine. Shave the site if necessary to remove non-vellus hairs. The site was cleansed with an alcohol wipe (70% isopropyl alcohol) and let air dry completely before application of the tape. The tape was then applied to the skin site. If more than one tape was used, application was in sequential order starting from the left side. A surgical skin marker and/or a water soluble marker was used to mark the location of the tape on the skin in order to align subsequent tapes.

The tape harvesting procedure was started by applying pressure to the tape and ensuring that the skin was held taut to ensure that the tape does not move while applying pressure. The tape was then removed slowly in one direction. An edge of the tape was then placed onto the strip at the top of a packet with the adhesive surface of the tape facing down to protect the sample. Sequential tapes were then put on the same site, if applicable, and removed slowly in an opposite direction to that used in the immediately previous application.

The sites of application were stripped with a total of at least four tapes, unless otherwise specified in the protocol. Tapes were then put into a storage bag and immediately placed on dry ice or into storage at -20°C or below until analysis.

Recent work by Benson *et al* (2006) demonstrates that RNA can be recovered from psoriatic lesions and that the general RNA expression profile of tape strip recovered RNA is consistent with biopsy RNA derived from lesions on the same patient. Further work (see U.S. Pat. No. 7,183,057, incorporated herein by reference) has shown that psoriatic lesions can be sampled with tape during treatment with Enbrel and that strong correlations could be made between gene expression in week one of treatment and clinical response at weeks 4 and 8. This work further establishes the credentials of tape stripping for the recovery of physiologically relevant RNA from the surface of the skin.

**Table 1**

| No. | Gene | Description |
|---|---|---|
| 1 | 235859_at | Myeloid/lymphoid or mixed-lineage leukemia 3 |
| 2 | 200799_at | heat shock 70kDa protein 1A |
| 3 | 1561754_at | |
| 4 | 210734_x_at | MYC associated factor X |
| 5 | 208403_x_at | MYC associated factor X |
| 6 | 212195_at | Interleukin 6 signal transducer (gp130, oncostatin M receptor) |
| 7 | 218250_s_at | CCR4-NOT transcription complex, subunit 7 |
| 8 | 201305_x_at | acidic (leucine-rich) nuclear phosphoprotein 32 family, member B |
| 9 | 211858_x_at | GNAS complex locus |
| 10 | 1558733_at | zinc finger and BTB domain containing 38 |
| 11 | 225778_at | fucosyltransferase 1 (galactoside 2-alpha-L-fucosyltransferase) |
| 12 | 214656_x_at | myosin IC |
| 13 | 227797_x_at | Hypothetical protein dJ12208.2 |
| 14 | 222404_x_at | butyrate-induced transcript 1 |
| 15 | 1559950_at | hypothetical LOC401449 |
| 16 | 207332_s_at | transferrin receptor (p90, CD71) |
| 17 | 225345_s_at | F-box protein 32 |
| 18 | 210904_s_at | interleukin 13 receptor, alpha 1 |
| 19 | 213002_at | Myristoylated alanine-rich protein kinase C substrate |
| 20 | 201670_s_at | myristoylated alanine-rich protein kinase C substrate |
| 21 | 211945_s_at | integrin, beta 1 (fibronectin receptor, beta polypeptide, antigen CD29 includes MDF2, MSK12) |
| 22 | 201294_s_at | WD repeat and SOCS box-containing 1 |
| 23 | 204083_s_at | tropomyosin 2 (beta) |
| 24 | 208658_at | protein disulfide isomerase family A, member 4 |
| 25 | 201635_s_at | fragile X mental retardation, autosomal homolog 1 |
| 26 | 204688_at | sarcoglycan, epsilon |
| 27 | 218045_x_at | parathymosin |
| 28 | 209715_at | chromobox homolog 5 (HP1 alpha homolog, Drosophila) |
| 29 | 220154_at | dystonin |
| 30 | 226400_at | Cell division cycle 42 (GTP binding protein, 25kDa) |
| 31 | 200748_s_at | ferritin, heavy polypeptide 1 |
| 32 | 211628_x_at | ferritin, heavy polypeptide pseudogene 1 ; ferritin, heavy polypeptide pseudogene 1 |
| 33 | 224559_at | metastasis associated lung adenocarcinoma transcript 1 (non-coding RNA) |
| 34 | 211452_x_at | leucine rich repeat (in FLII) interacting protein 1 |
| 35 | 214316_x_at | Calreticulin |
| 36 | 238427_at | GrpE-like 2, mitochondrial (E. coli) |
| 37 | 208637_x_at | actinin, alpha 1 |
| 38 | 200800_s_at | heat shock 70kDa protein 1A ; heat shock 70kDa protein 1B |
| 39 | 225767_at | hypothetical protein LOC284801 |
| 40 | 1565525_a_at | t-complex 11 (mouse) like 2 |
| 41 | 224625_x_at | small EDRK-rich factor 2 |
| 42 | 217756_x_at | small EDRK-rich factor 2 |
| 43 | 215904_at | myeloid/lymphoid or mixed-lineage leukemia (trithorax homolog, Drosophila); translocated to, 4 |
| 44 | 1558048_x_at | |
| 45 | 201631_s_at | immediate early response 3 |
| 46 | 217137_x_at | Kpni repeat mrna (cdna clone pcd-kpni-8), 3' end |
| 47 | 224153_s_at | |
| 48 | 217202_s_at | glutamate-ammonia ligase (glutamine synthetase) |
| 49 | 214279_s_at | NDRG family member 2 |
| 50 | 214278_s_at | NDRG family member 2 |
| 51 | 202289_s_at | transforming, acidic coiled-coil containing protein 2 |
| 52 | 236009_at | Transcribed locus |
| 53 | 207320_x_at | staufen, RNA binding protein (Drosophila) |
| 54 | 208640_at | ras-related C3 botulinum toxin substrate 1 (rho family, small GTP binding protein Rac1) |
| 55 | 211383_s_at | WD repeat domain 37 |
| 56 | 200840_at | lysyl-tRNA synthetase |
| 57 | 200042_at | hypothetical protein HSPC117 ; hypothetical protein HSPC117 |
| 58 | 221532_s_at | WD repeat domain 61 |
| 59 | 203983_at | translin-associated factor X |
| 60 | 225176_at | MSTP146 (MST146) |
| 61 | 214131_at | chromosome Y open reading frame 15B |
| 62 | 204409_s_at | eukaryotic translation initiation factor 1A, Y-linked |
| 63 | 201909_at | ribosomal protein S4, Y-linked 1 |
| 64 | 225540_at | Microtubule-associated protein 2 |
| 65 | 223279_s_at | uveal autoantigen with coiled-coil domains and ankyrin repeats |
| 66 | 225846_at | RNA binding motif protein 35A |
| 67 | 204571_x_at | protein (peptidyl-prolyl cis/trans isomerase) NIMA-interacting, 4 (parvulin) |
| 68 | 204060_s_at | protein kinase, X-linked ; protein kinase, Y-linked |
| 69 | 218109_s_at | major facilitator superfamily domain containing 1 |
| 70 | 202088_at | solute carrier family 39 (zinc transporter), member 6 |
| 71 | 228259_s_at | Erythrocyte membrane protein band 4.1 like 4A |
| 72 | 202020_s_at | LanC lantibiotic synthetase component C-like 1 (bacterial) |
| 73 | 212929_s_at | family with sequence similarity 21, member B ; family with sequence similarity 21, member C ; similar to KIAA0592 protein ; similar to KIAA0592 protein |
| 74 | 217846_at | glutaminyl-tRNA synthetase |
| 75 | 213728_at | lysosomal-associated membrane protein I |
| 76 | 211755_s_at | ATP synthase, H+ transporting, mitochondrial F0 complex, subunit b, isoform ATP synthase, H+ transporting, mitochondrial F0 complex, subunit b, isoform 1 |
| 77 | 217724_at | SERPINE1 mRNA binding protein 1 |
| 78 | 217900_at | isoleucine-tRNA synthetase 2, mitochondrial |
| 79 | 222980_at | RAB10, member RAS oncogene family |
| 80 | 228520_s_at | Amyloid beta (A4) precursor-like protein 2 |
| 81 | 217773_s_at | NADH dehydrogenase (ubiquinone) 1 alpha subcomplex, 4, 9kDa |
| 82 | 217940_s_at | hypothetical protein FLJ10769 |
| 83 | 214431_at | guanine monphosphate synthetase |
| 84 | 218403_at | p53-inducible cell-survival factor |
| 85 | 209212_s_at | Kruppel-like factor 5 (intestinal) |
| 86 | 209211_at | Kruppel-like factor 5 (intestinal) |
| 87 | 223302_s_at | zinc finger protein 655 |
| 88 | 209135_at | aspartate beta-hydroxylase |
| 89 | 233080_s_at | formin binding protein 3 |
| 90 | 208704_x_at | amyloid beta (A4) precursor-like protein 2 |
| 91 | 208248_x_at | amyloid beta (A4) precursor-like protein 2 |
| 92 | 213194_at | roundabout, axon guidance receptor, homolog 1 (Drosophila) |
| 93 | 231896_s_at | density-regulated protein |
| 94 | 228538_at | zinc finger protein 662 |
| 95 | 224650_at | mal, T-cell differentiation protein 2 |
| 96 | 204256_at | ELOVL family member 6, elongation of long chain fatty acids (FEN1/Elo2, SUR4/Elo3-like, yeast) |
| 97 | 201553_s_at | lysosomal-associated membrane protein 1 |
| 98 | 202594_at | leptin receptor overlapping transcript-like 1 |
| 99 | 229017_s_at | receptor interacting protein kinase 5 |
| 100 | 227717_at | FLJ41603 protein |

**Table 2**

| Class | No. | Gene | Description |
|---|---|---|---|
| **"Old"** | 1 | 202227_s_at | bromodomain containing 8 |
| | 2 | 201024_x_at | eukaryotic translation initiation factor 5B |
| | 3 | 228360_at | hypothetical protein LOC130576 |
| | 4 | 214314_s_at | eukaryotic translation initiation factor 5B |
| | 5 | 200842_s_at | glutamyl-prolyl-tRNA synthetase |
| | 6 | 213136_at | protein tyrosine phosphatase, non-receptor type 2 |
| | 7 | 36129_at | RUN and TBC1 domain containing 1 |
| | 8 | 237626_at | RB1-inducible coiled-coil 1 |
| | 9 | 233303_at | Threonine synthase, chloroplast |
| | 10 | 213387_at | KIAA1240 protein |
| | 11 | 238408_at | Oxidation resistance 1 |
| | 12 | 241245_at | Splicing factor, arginine/serine-rich 4 |
| | 13 | 1557012_a_at | CDNA clone IMAGE:4816709 |
| | 14 | 232406_at | Jagged 1 (Alagille syndrome) |
| | 15 | 228103_s_at | Neuropilin 2 |
| | 16 | 201685_s_at | chromosome 14 open reading frame 92 |
| | 17 | 210319_x_at | msh homeo box homolog 2 (Drosophila) |
| | 18 | 222513_s_at | sorbin and SH3 domain containing 1 |
| | 19 | 225988_at | hect domain and RLD 4 |
| | 20 | 239203_at | hypothetical protein FLJ39575 |
| **"Young"** | 21 | 211467_s_at | nuclear factor I/B |
| | 22 | 213029_at | Nuclear factor I/B |
| | 23 | 226614_s_at | chromosome 8 open reading frame 13 |
| | 24 | 201365_at | ornithine decarboxylase antizyme 2 |
| | 25 | 218062_x_at | CDC42 effector protein (Rho GTPase binding) 4 |
| | 26 | 222404_x_at | butyrate-induced transcript 1 |
| | 27 | 215707_s_at | prion protein (p27-30) (Creutzfeld-Jakob disease, Gerstmann-Strausler-Scheinker syndrome, fatal familial insomnia) |
| | 28 | 226835_s_at | transaldolase 1 ; similar to RPE-spondin |
| | 29 | 209109_s_at | tetraspanin 6 |
| | 30 | 39249_at | aquaporin 3 |
| | 31 | 210734_x_at | MYC associated factor X |
| | 32 | 210125_s_at | barrier to autointegration factor 1 |
| | 33 | 218143_s_at | secretory carrier membrane protein 2 |
| | 34 | 207332_s_at | transferrin receptor (p90, CD71) |
| | 35 | 202731_at | programmed cell death 4 (neoplastic transformation inhibitor) |
| | 36 | 202730_s_at | programmed cell death 4 (neoplastic transformation inhibitor) |
| | 37 | 203126_at | inositol(myo)-1(or 4)-monophosphatase 2 |
| | 38 | 200862_at | 24-dehydrocholesterol reductase |
| | 39 | 214091_s_at | glutathione peroxidase 3 (plasma) |
| | 40 | 201348_at | glutathione peroxidase 3 (plasma) |
| | 41 | 214687_x_at | aldolase A, fructose-bisphosphate |
| | 42 | 221764_at | chromosome 19 open reading frame 22 |
| | 43 | 215243_s_at | gap junction protein, beta 3, 31kDa (connexin 31) |
| | 44 | 205490_x_at | gap junction protein, beta 3, 31kDa (connexin 31) |
| | 45 | 225177_at | RAB 11 family interacting protein 1 (class I) |
| | 46 | 41858_at | FGF receptor activating protein 1 |
| | 47 | 209173_at | anterior gradient 2 homolog (Xenopus laevis) |
| | 48 | 229013_at | LOC440282 |
| | 49 | 201888_s_at | interleukin 13 receptor, alpha 1 |
| | 50 | 227475_at | forkhead box Q¹ |
| | 51 | 214279_s_at | NDRG family member 2 |
| | 52 | 229004_at | ADAM metallopeptidase with thrombospondin type 1 motif, 15 |
| | 53 | 208792_s_at | clusterin (complement lysis inhibitor, SP-40,40, sulfated glycoprotein 2, testosterone-repressed prostate message 2, apolipoprotein J) |
| | 54 | 205470_s_at | kallikrein 11 |
| | 55 | 205783_at | kallikrein 13 |
| | 56 | 209792_s_at | kallikrein 10 |
| | 57 | 204733_at | kallikrein 6 (neurosin, zyme) |
| | 58 | 1552620_at | small proline rich protein 4 |
| | 59 | 214549_x_at | small proline-rich protein 1A |
| | 60 | 213796_at | small proline-rich protein 1A |
| | 61 | 232729_at | F-box protein 32 |

**Table 3**

| Class | No. | Gene | description |
|---|---|---|---|
| **"Young"** | 1 | 202054_s_at | aldehyde dehydrogenase 3 family, member A2 |
| | 2 | 202053_s_at | aldehyde dehydrogenase 3 family, member A2 |
| | 3 | 210544_s_at | aldehyde dehydrogenase 3 family, member A2 |
| | 4 | 211467_s_at | nuclear factor I/B |
| | 5 | 213029_at | Nuclear factor I/B |
| | 6 | 209290_s_at | nuclear factor I/B |
| | 7 | 209289_at | Nuclear factor I/B |
| | 8 | 1552703_s_at | caspase 1, apoptosis-related cysteine peptidase (interleukin 1, beta, convertase); caspase-1 dominant-negative inhibitor pseudo-ICE |
| | 9 | 225911_at | nephronectin |
| | 10 | 217722_s_at | neugrin, neurite outgrowth associated |
| | 11 | 218062_x_at | CDC42 effector protein (Rho GTPase binding) 4 |
| | 12 | 224570_s_at | interferon regulatory factor 2 binding protein 2 |
| | 13 | 224571_at | interferon regulatory factor 2 binding protein 2 |
| | 14 | 233814_at | CDNA: FLJ22256 fis, clone HRC02860 |
| | 15 | 213032_at | Nuclear factor I/B |
| | 16 | 230291_s_at | Nuclear factor I/B |
| | 17 | 213033_s_at | Nuclear factor I/B |
| | 18 | 226465_s_at | SON DNA binding protein |
| | 19 | 200906_s_at | palladin |
| | 20 | 200897_s_at | palladin |
| | 21 | 201286_at | syndecan 1 |
| | 22 | 202712_s_at | creatine kinase, mitochondrial 1B ; creatine kinase, mitochondrial 1A |
| | 23 | 223601_at | olfactomedin 2 |
| | 24 | 210734_x_at | MYC associated factor X |
| | 25 | 208403_x_at | MYC associated factor X |
| | 26 | 218045_x_at | parathymosin |
| | 27 | 204306_s_at | CD 151 antigen |
| | 28 | 200621_at | cysteine and glycine-rich protein 1 |
| | 29 | 202592_at | biogenesis of lysosome-related organelles complex-1, subunit 1 |
| | 30 | 206140_at | LIM homeobox 2 |
| | 31 | 203921_at | carbohydrate (N-acetylglucosamine-6-O) sulfotransferase 2 |
| | 32 | 217897_at | FXYD domain containing ion transport regulator 6 |
| | 33 | 227317_at | LIM and cysteine-rich domains 1 |
| | 34 | 212082_s_at | myosin, light polypeptide 6, alkali, smooth muscle and non-muscle |
| | 35 | 213214_x_at | actin, gamma 1 |
| | 36 | 224585_x_at | actin, gamma 1 |
| | 37 | 212988_x_at | actin, gamma 1 |
| | 38 | 211970_x_at | actin, gamma 1 |
| | 39 | 211985_s_at | calmodulin 1 (phosphorylase kinase, delta) |
| | 40 | 203752_s_at | jun D proto-oncogene |
| | 41 | 206453_s_at | NDRG family member 2 |
| | 42 | 202935_s_at | SRY (sex determining region Y)-box 9 (campomelic dysplasia, autosomal sex-reversal) |
| | 43 | 202936_s_at | SRY (sex determining region Y)-box 9 (campomelic dysplasia, autosomal sex-reversal) |
| | 44 | 200762_at | dihydropyrimidinase-like 2 |
| | 45 | 209118_s_at | tubulin, alpha 3 |
| | 46 | 201556_s_at | vesicle-associated membrane protein 2 (synaptobrevin 2) |
| | 47 | 202575_at | cellular retinoic acid binding protein 2 |
| | 48 | 229004_at | ADAM metallopeptidase with thrombospondin type 1 motif, 15 |
| | 49 | 228993_s_at | hypothetical protein LOC92482 |
| | 50 | 212593_s_at | programmed cell death 4 (neoplastic transformation inhibitor) |
| | 51 | 202730_s_at | programmed cell death 4 (neoplastic transformation inhibitor) |
| | 52 | 202731_at | programmed cell death 4 (neoplastic transformation inhibitor) |
| | 53 | 212594_at | programmed cell death 4 (neoplastic transformation inhibitor) |
| | 54 | 39248_at | aquaporin 3 |
| | 55 | 201631_s_at | immediate early response 3 |
| | 56 | 205249_at | early growth response 2 (Krox-20 homolog, Drosophila) |
| | 57 | 201464_x_at | v-jun sarcoma virus 17 oncogene homolog (avian) |
| | 58 | 200965_s_at | actin binding LIM protein 1 |
| | 59 | 225615_at | hypothetical protein LOC126917 |
| | 60 | 212377_s_at | Notch homolog 2 (Drosophila) |
| | 61 | 202443_x_at | Notch homolog 2 (Drosophila) |
| | 62 | 226614_s_at | chromosome 8 open reading frame 13 |
| | 63 | 205157_s_at | keratin 17 |
| | 64 | 212236_x_at | keratin 17 |
| | 65 | 223449_at | sema domain, transmembrane domain (TM), and cytoplasmic domain, (semaphorin) 6A |
| | 66 | 204254_s_at | vitamin D (1,25- dihydroxyvitamin D3) receptor |
| | 67 | 229013_at | LOC440282 |
| | 68 | 225345_s_at | F-box protein 32 |
| | 69 | 228922_at | Src homology 2 domain containing F |
| | 70 | 226899_at | unc-5 homolog B (C. elegans) |
| | 71 | 1556839_s_at | Spectrin, beta, non-erythrocytic 5 |
| | 72 | 1553602_at | small breast epithelial mucin |
| | 73 | 209792_s_at | kallikrein 10 |
| | 74 | 213796_at | small proline-rich protein 1A |
| | 75 | 1552620_at | small proline rich protein 4 |
| | 76 | 206595_at | cystatin E/M |
| | 77 | 213680_at | keratin 6B |
| | 78 | 203315_at | NCK adaptor protein 2 |
| | 79 | 233641_s_at | Chromosome 8 open reading frame 13 |
| | 80 | 202341_s_at | tripartite motif-containing 2 |
| | 81 | 228575_at | fibronectin type III domain containing 6 |
| | 82 | 201161_s_at | cold shock domain protein A |
| | 83 | 200696_s_at | gelsolin (amyloidosis, Finnish type) |
| | 84 | 209126_x_at | keratin 6B |
| | 85 | 225035_x_at | CXYorf1-related protein ; CXYorf1-related protein ; CXYorf1-related protein |
| | 86 | 208864_s_at | thioredoxin |
| **"Old"** | 87 | 231925_at | CDNA: FLJ23006 fis, clone LNG00414 |
| | 88 | 219756_s_at | premature ovarian failure, 1B |
| | 89 | 201926_s_at | decay accelerating factor for complement (CD55, Cromer blood group system) |
| | 90 | 201925_s_at | decay accelerating factor for complement (CD55, Cromer blood group system) |
| | 91 | 203691_at | peptidase inhibitor 3, skin-derived (SKALP) ; peptidase inhibitor 3, skin-derived (SKALP) |
| | 92 | 41469_at | peptidase inhibitor 3, skin-derived (SKALP) |
| | 93 | 218963_s_at | keratin 23 (histone deacetylase inducible) |
| | 94 | 236119_s_at | small proline-rich protein 2G |
| | 95 | 242204_at | WAP four-disulfide core domain 5 |
| | 96 | 206177_s_at | arginase, liver |
| | 97 | 207381_at | arachidonate 12-lipoxygenase, 12R type |
| | 98 | 203575_at | casein kinase 2, alpha prime polypeptide |
| | 99 | 215380_s_at | chromosome 7 open reading frame 24 |
| | 100 | 207908_at | keratin 2A (epidermal ichthyosis bullosa of Siemens) |
| | 101 | 237563_s_at | LOC440731 |
| | 102 | 225239_at | CDNA FLJ26120 fis, clone SYN00419 |
| | 103 | 238320_at | trophoblast-derived noncoding RNA |
| | 104 | 220983_s_at | sprouty homolog 4 (Drosophila) ; sprouty homolog 4 (Drosophila) |
| | 105 | 236266_at | CDNA FLJ31407 fis, clone NT2NE2000137 |
| | 106 | 202179_at | bleomycin hydrolase |

Although the invention has been described with reference to the above examples, it will be understood that modifications and variations are encompassed within the spirit and scope of the invention. Accordingly, the invention is limited only by the following claims.

### Embodiments of the Invention

Paragraph 1. A method for determining the age range of a skin sample of a subject comprising analyzing nucleic acid from one or more genes listed in Table 1, Table 2, Table 3, or any combination thereof, in a sample of the skin from the subject, thereby determining the age range of the skin of the subject.
Paragraph 2. The method of paragraph 1 , wherein the nucleic acid is RNA.
Paragraph 3. The method of paragraph 1, wherein analyzing the nucleic acid comprises detecting one or more mutations in the nucleic acid sequence of the nucleic acid.
Paragraph 4. The method of paragraph 3, wherein the one or more mutations are selected from the group consisting of a substitution, a deletion, and an insertion.
Paragraph 5. The method of paragraph 1 , further comprising amplifying the nucleic acid obtained from the sample prior to analyzing.
Paragraph 6. The method of paragraph 1, wherein the sample is obtained by applying an adhesive tape to a target area of skin in a manner sufficient to isolate the sample adhering to the adhesive tape.
Paragraph 7. The method of paragraph 1, further comprising using the characterizing to determine a treatment regimen for the symptoms of photoaging or chronoaging of the subject.
Paragraph 8. The method of paragraph 3, wherein the isolated nucleic acid or an amplification product thereof, is applied to a microarray.
Paragraph 9. The method of paragraph 8, wherein an expression profile is detected using a microarray.
Paragraph 10. The method of paragraph 1 , wherein the sample is obtained from a biopsy taken at the site of the skin lesion or surrounding margin.
Paragraph 11. The method of paragraph 6, wherein the tape comprises a rubber adhesive on a polyurethane film.
Paragraph 12. The method of paragraph 6, wherein about one to ten adhesive tapes or one to ten applications of a tape are applied and removed from the skin.
Paragraph 13. The method of paragraph 6, wherein about one to eight adhesive tapes or one to eight applications of a tape are applied and removed from the skin.
Paragraph 14. The method of paragraph 6, wherein about one to five adhesive tapes or one to five applications of a tape are applied and removed from the skin.
Paragraph 15. The method of paragraph 6, wherein the method further comprises taking a biopsy of the target area of the skin.
Paragraph 16. The method of paragraph 3, wherein the analyzing is performed in situ.
Paragraph 17. A method of identifying an age range for a skin sample comprising analyzing a nucleic acid from the sample as compared with one or more genes listed in Table 1, Table 2, Table 3, or any combination thereof, and identifying the age range of the skin based on the analysis.
Paragraph 18. The method of paragraph 17, wherein the nucleic acid is RNA.
Paragraph 19. The method of paragraph 17, wherein analyzing the nucleic acid comprises detecting one or more mutations in the nucleic acid sequence of the nucleic acid.
Paragraph 20. The method of paragraph 19, wherein the one or more mutations are selected from the group consisting of a substitution, a deletion, and an insertion.
Paragraph 21. The method of paragraph 17, further comprising amplifying the nucleic acid obtained from the sample prior to analyzing.
Paragraph 22. The method of paragraph 17, wherein the sample is obtained by applying an adhesive tape to a target area of skin in a manner sufficient to isolate the sample adhering to the adhesive tape, wherein the sample comprises nucleic acid molecules.
Paragraph 23. The method of paragraph 19, wherein the isolated nucleic acid or an amplification product thereof, is applied to a microarray.
Paragraph 24. The method of paragraph 19, wherein an expression profile is detected using a microarray.
Paragraph 25. The method of paragraph 17, wherein the sample is obtained from a biopsy taken from a target area of skin from the subject.
Paragraph 26. The method of paragraph 22, wherein the tape comprises a rubber adhesive on a polyurethane film.
Paragraph 27. The method of paragraph 22, wherein about one to ten adhesive tapes or one to ten applications of a tape are applied and removed from the skin.
Paragraph 28. The method of paragraph 22, wherein about one to eight adhesive tapes or one to eight applications of a tape are applied and removed from the skin.
Paragraph 29. The method of paragraph 22, wherein about one to five adhesive tapes or one to five applications of a tape are applied and removed from the skin.
Paragraph 30. The method of paragraph 22, wherein the method further comprises taking a biopsy of the target area of the skin.
Paragraph 31. The method of paragraph 19, wherein the analyzing is performed in situ.
Paragraph 32. A method for determining the age range of skin of a subject comprising detecting an altered level of a target protein in a sample from the subject, as compared to the level of the target protein in a corresponding sample from a subject of known age range, wherein the protein is an expression product of a gene listed in Table 1, Table 2, Table 3, or any combination thereof, thereby determining the age range of the subject.
Paragraph 33. The method of paragraph 32, wherein the sample is obtained by applying an adhesive tape to a target area of skin in a manner sufficient to isolate the sample adhering to the adhesive tape, wherein the sample comprises cells, and further comprising lysing the cells to extract the target protein.
Paragraph 34. The method of paragraph 33, wherein the tape comprises a rubber adhesive on a polyurethane film.
Paragraph 35. The method of paragraph 33, wherein between one and ten adhesive tapes are applied to the skin and removed from the skin.
Paragraph 36. The method of paragraph 33, wherein about one to eight adhesive tapes are applied and removed from the skin.
Paragraph 37. The method of paragraph 33, wherein about one to five adhesive tapes are applied and removed from the skin.
Paragraph 38. The method of paragraph 33, wherein the method further comprises taking a biopsy of the target area of the skin.
Paragraph 39. The method of paragraph 38, wherein protein is extracted from the biopsy sample, and the level of protein in the biopsy and the level of protein in the tape sample are analyzed.
Paragraph 40. The method of paragraph 32, wherein the sample is obtained by from a biopsy of a target area of skin.
Paragraph 41. A method for determining the age range of skin of a subject comprising:
   a) providing a gene expression profile of a target area of the skin of the subject, wherein the target area of the skin simultaneously expresses a plurality of genes at the protein level that are markers for a specific age range; and
   b) comparing the subject's gene expression profile to a reference gene expression profile obtained from a corresponding skin sample of known age range, wherein the reference gene expression profile comprises an expression value of one or more target genes listed in Table 1, Table 2, Table 3, or any combination thereof.
Paragraph 42. The method of paragraph 41 , wherein the reference gene expression profile is contained within a database.
Paragraph 43. The method of paragraph 41 , wherein the comparing is carried out using a computer algorithm.
Paragraph 44. A kit for characterizing a skin sample from a subject comprising a skin sample collection device and one or more probes or primers that selectively bind to one or more nucleic acid molecules in Table 1, Table 2, Table 3, or any combination thereof, or to a nucleic acid or protein product of a nucleic acid molecule in Table 1, Table 2, Table 3, or any combination thereof.
Paragraph 45. The kit of paragraph 44, wherein the kit provides a probe which binds to a portion of a nucleic acid molecule in Table 1, Table 2, Table 3, or any combination thereof.
Paragraph 46. The kit of paragraph 44, wherein the kit provides one or more primer pairs comprising a forward primer that selectively binds upstream of a gene on one strand and a reverse primer that selectively binds upstream of a gene on a complementary strand, wherein the gene is any gene listed in Table 1, Table 2, Table 3, or any combination thereof. Paragraph 47. The kit of paragraph 44, wherein the skin sample collection device is a biopsy needle.
Paragraph 48. The kit of paragraph 44, wherein the skin sample collection device is an adhesive tape.
Paragraph 49. The kit of paragraph 48, wherein the adhesive tape comprises a rubber adhesive on a polyurethane film.
Paragraph 50. The kit of paragraph 44, further comprising a microarray containing at least a fragment of a gene or a nucleic acid or protein product of any gene listed in Table 1, Table 2, Table 3, or any combination thereof.
Paragraph 51. A kit for determining the age range of skin in a subject comprising an applicator and one or more probes or primers that selectively bind to one or more of nucleic acid molecules in Table 1, Table 2, Table 3, or any combination thereof, or to a nucleic acid or protein expression product of a nucleic acid molecule in any of Table 1, Table 2, Table 3, or any combination thereof.
Paragraph 52. The kit of paragraph 51 , wherein the probes are detectably labeled.
Paragraph 53. The method of any one of paragraphs 1, 17, 32, or 41, wherein the subject is human.
Paragraph 54. The method of any one of paragraphs 1, 17, 32, or 41, wherein the sample is an epidermal sample.
Paragraph 55. A cosmetic formulation containing agents for reducing or increasing expression of genes in Table 1, Table 2, Table 3, or any combination thereof.
Paragraph 56. The cosmetic formulation of paragraph 55, wherein the cosmetic formulation comprises an emulsion, a cream, a lotion, a solution, an anhydrous base, a paste, a powder, a gel, or an ointment.
Paragraph 57. The cosmetic formulation of paragraph 56, wherein the emulsion is an oil-in-water emulsion or a water-in-oil emulsion.
Paragraph 58. The cosmetic formulation of paragraph 55, wherein the cosmetic formulation is a solution, and wherein the solution is an aqueous solution or hydro-alcoholic solution.
Paragraph 59. The cosmetic formulation of paragraph 55, wherein the cosmetic formulation is an anhydrous base, and wherein the anhydrous base is a lipstick or a powder.
Paragraph 60. The cosmetic formulation of paragraph 55, wherein the formulation is comprised in an anti-aging product or a moisturizing product.
Paragraph 61. The cosmetic formulation of paragraph 55, wherein the formulation further comprises one or more of estradiol; progesterone; pregnanalone; coenzyme QIO; methylsolanomethane (MSM); copper peptide (copper extract); plankton extract (phytosome); glycolic acid; kojic acid; ascorbyl palmitate; all trans retinol; azaleic acid; salicylic acid; broparoestrol; estrone; adrostenedione; and androstanediols.
Paragraph 62. The cosmetic formulation of paragraph 55, wherein the formulation further comprises a sunblock.

## Claims

1. A method for determining the age range of a skin sample comprising:
a) providing a skin sample comprising nucleic acids expressed by genes in a classifier comprising one or more young genes and one or more old genes;
b) determining expression levels of the nucleic acids expressed from at least one young gene and at least one old gene in the classifier by assaying the nucleic acids in the skin sample; and
c) characterizing the age range of the skin sample based on the expression levels.

2. The method of claim 1, wherein the nucleic acids comprise RNA.

3. The method of claim 2, wherein the method comprises generating cDNA molecules from the RNA.

4. The method of claim 3, wherein assaying the nucleic acids in the skin sample comprises amplification of the cDNA molecules using real-time quantitative polymerase chain reaction (PCR).

5. The method of claim 1, wherein determining the expression levels comprises real-time quantitative PCR, microarray analysis, or a direct sequencing method.

6. The method of claim 1, wherein the sample is obtained by applying an adhesive tape to a target area of skin in a manner sufficient to isolate the sample adhering to the adhesive tape.

7. The method of claim 6, wherein the tape comprises a rubber adhesive on a polyurethane film.

8. The method of claim 7, wherein about one to about ten adhesive tapes or about one to about ten applications of a tape are applied to and removed from the skin.

9. The method of claim 1, wherein characterizing the age range of the skin sample comprises comparing the expression levels of the nucleic acids expressed from the at least one young gene and the at least one old gene in the skin sample to a reference expression profile.

10. The method of claim 9, wherein the reference expression profile comprises known expression levels of nucleic acids expressed from one or more genes in the classifier in one or more reference samples.

11. The method of claim 10, wherein the reference expression profile is contained within a database.

12. The method of claim 9, wherein the comparing is carried out using a computer algorithm.

13. The method of claim 10, wherein the one or more reference samples comprises a sample obtained from a subject with young skin, a sample obtained from a subject with old skin, or a combination thereof.

14. A kit for characterizing a skin sample of a subject comprising
a) a skin sample collection device; and
b) one or more probes, one or more primers, or a combination thereof, for use in detecting expression of a nucleic acid from at least one young gene and at least one old gene in a gene classifier.

15. The kit of claim 14, wherein the skin sample collection device is an adhesive tape.
